# EUROPÄISCHE PATENTANMELDUNG

(11) **EP 2 105 500 A1**
(43) Veröffentlichungstag der Anmeldung: **30.09.2009**
(21) Anmeldenummer: 08153330.9
(22) Anmeldetag: 26.03.2008
(51) Int. Cl.: C12N 9/04

(54) **Neue 12alpha-Hydroxysteroiddehydrogenasen, deren Herstellung und deren Verwendung**

(71) Anmelder: Pharmazell GmbH, 83064 Raubling (DE)
(72) Erfinder: Schmid, Rolf, 70569 Stuttgart (DE); Braun, Michael, 97980 Bad Mergentheim-Edelfingen (DE); Maurer, Steffen, 67246 Dirmstein (DE)
(74) Vertreter: Reitstötter - Kinzebach

(57) **Zusammenfassung**

Die vorliegende Erfindung betrifft neue 12α-Hydroxysteroiddehydrogenasen, dafür kodierende Nukleinsäuresequenzen, Expressionskassetten und Vektoren; rekombinante Mikroorganismen, enthaltend entsprechende kodierende Nukleinsäuresequenzen; Verfahren zur Herstellung solcher 12 α-Hydroxysteroiddehydrogenasen; Verfahren zurenzymatischen Oxidation von 12α-Hydroxysteroiden unter Verwendung derartiger Enzyme, Verfahren zur enzymatischen Reduktion von 12-Ketosteroiden unter Verwendung solcher Enzyme; Verfahren zur qualitativen oder quantitativen Bestimmung von 12-Ketosteroiden bzw. 12α-Hydroxysteroiden unter Verwendung der erfindungsgemäßen 12α-Hydroxysteroiddehydrogenasen; sowie ein Verfahren zur Herstellung von Ursodesoxycholsäure, umfassend die enzymkatalysierte Cholsäureoxidation unter Verwendung der erfindungsgemäßen 12α-Hydroxysteroiddehydrogenasen.

## Beschreibung

Die vorliegende Erfindung betrifft neue 12α-Hydroxysteroiddehydrogenasen, dafür kodierende Nukleinsäuresequenzen, Expressionskassetten und Vektoren; rekombinante Mikroorganismen, enthaltend entsprechende kodierende Nukleinsäuresequenzen; Verfahren zur Herstellung solcher 12 α-Hydroxysteroiddehydrogenasen; Verfahren zur enzymatischen Oxidation von 12α-Hydroxysteroiden unter Verwendung derartiger Enzyme, Verfahren zur enzymatischen Reduktion von 12-Ketosteroiden unter Verwendung solcher Enzyme; Verfahren zur qualitativen oder quantitativen Bestimmung von 12-Ketosteroiden bzw. 12α-Hydroxysteroiden unter Verwendung der erfindungsgemäßen 12α-Hydroxysteroiddehydrogenasen; sowie ein Verfahren zur Herstellung von Ursodesoxycholsäure, umfassend die enzymkatalysierte Cholsäureoxidation unter Verwendung der erfindungsgemäßen 12α-Hydroxysteroiddehydrogenasen.

### Hintergrund der Erfindung

Die 12α-Hydroxysteroiddehydrogenase (12α-HSDH) (E.C. 1.1.1.176) ist ein für die stereospezifische Synthese, wie z.B. die Oxidation von Cholsäure, wichtiger Biokatalysator.

Untersuchungen zu einer 12α-HSDH aus *Clostridium sp.* group P strain 48 - 50 und deren partielle Reinigung durch NAD⁺ und NADP⁺-Sepharose Chromatographie wurde von Mahony et al. (Mahony, D.E., et alAppl Environ Microbiol, 1977. 34(4): p. 419-23) bzw. Macdonald et al, (Macdonald, I.A., et al, Journal of Lipid Research, 1979, 20 234-239) beschrieben.

Ein entsprechend hergestellter 12α-HSDH-haltiger Proteinextrakt aus *Clostridium* Gruppe P wurde von Sutherland et al. im Rahmen von drei verschiedenen Synthesewegen von Ursodesoxycholsäure aus Cholsäure eingesetzt. Einer der Synthesewege umfasste dabei die enzymkatalysierte Oxidation von Cholsäure zu 12-Ketochenodesoxycholsäure (12-Keto-CDCS) (Sutherland, J.D., et al, Prep Biochem, 1982. 12(4): p. 307-21). Als Enzympräparation kam dabei Zelllysat aus *Clostridium sp.* group P strain 48 -50 DSM 4029 zum Einsatz. Die Reaktion benötigt stöchiometrische Mengen des Kofaktors NADP⁺.

Der Bedarf an Kofaktor kann durch die Kopplung mit einem kofaktorregenerierenden Enzym verringert werden. Der Stand der Technik lehrt hierzu beispielsweise die Verwendung von Glutamatdehydrogenase (GLDH), welche zusammen mit einem 12α-HSDH-haltigen Proteinextrakt *Clostridium* Gruppe P coimmobilisiert ist (Carrea, G., et al., Biotechnology and Bioengineering, 1984. 26(5): p. 560-563). Die GLDH reoxidiert NADPH zu NADP⁺ unter gleichzeitiger reduktiver Aminierung von α-Ketoglutarat zu Glutamat. Alternativ dazu werden auch Alkoholdehydrogenasen ADH-Tb, ADH-Lb und ADH-ms zur Kofaktorregenerierung vorgeschlagen (Fossati, E., et al., Biotechnol Bioeng, 2006. 93(6): p. 1216-20). Die ADH wandelt Aceton in 2-Propanol unter Regenerierung von NADP⁺ um. Für die Umsetzung im 10-ml Maßstab wurde dort wiederum kein Reinprotein, sondern eine aus einem kommerziellen Präparat (von ASA Spezialenzyme, Wolfenbüttel, Deutschland) weiter angereicherte 12α-HSDH-haltigen Proteinfraktion mit einer spezifischen Aktivität von 12 U/mg Protein eingesetzt.

Allen oben beschriebenen Untersuchungen ist gemeinsam, dass die Quelle für 12α-HSDH der pathogene und anaerobe Stamm *Clostridium sp.* group P strain 48 -50 ist. Aufgrund des geringen Anteils dies Enzyms am Gesamtprotein (max 1 % des Gesamtproteins von *Clostridium sp.*) wird einerseits der Zugang zu technisch nutzbaren Mengen des Enzyms erschwert. Andererseits gestaltet sich dessen industrielle Produktion aufwändig und kostspielig, da die gesamte Kultivierung und Lagerung des pathogenen Produktionsstamms in einem Betrieb durchgeführt werden muss, der die Zulassung für mikrobiologische Arbeiten der Risikogruppe 2 besitzt (siehe BioStoffV).

Zusätzlich macht die Pathogenität dieses Produktionsstamms den Einsatz von 12α-HSDH in der Synthese eines pharmazeutischen Zwischenprodukts problematisch. Für die Zulassung des Produktionsprozesses nach GMP-Regularien ist eine nicht-pathogene Enzymquelle erforderlich.

Die von einer NAD⁺-abhängigen 12α-HSDH katalysierte Cholsäureoxidation mit Kofaktorregenerierung durch Lactatdehydrogenase (LDH; Umsetzung von Pyruvat zu Lactat unter Regenerierung von NAD⁺) wird in der EP-A-1 731 618 beschrieben.

Weiterhin wurde eine zweistufige Aufreinigungsstrategie, basierend auf einer Farbstoffsäulen-Affinitätschromatographie für das Wildtyp-Enzym aus *Clostridium sp.* group P strain 48 -50 und eine N-terminale Aminosäuresequenz vorgeschlagen (Braun, M., et al., Eur J Biochem, 1991. 196(2): p. 439-50). Als N-terminale Sequenz wurde eine 29 Aminosäurereste umfassende Teilsequenz publiziert, deren N-Terminus wie folgt lautet:: Met-Ile-Phe-Asp-Gly-Lys-Val...... Zudem wurden von dieser Arbeitsgruppe keine kommerziell erhältlichen Säulenmaterialien für die Aufreinigung verwendet.

Derzeit wird ein 12α-HSDH enthaltender Extrakt aus *Clostridium sp.* group P strain 48 - 50 von ASA Spezialenzyme, Wolfenbüttel, Deutschland vertrieben. Untersuchungen zeigen jedoch, dass die geringe spezifische Aktivität dieses kommerziellen Präparats die Extraktion und Aufarbeitung von Reaktionsprodukten der 12α-HSDH-katalysierten enzymatischen Umsetzungen wegen der einzusetzenden hohen Gesamtproteinmenge erschwert.

Es ist daher Aufgabe der vorliegenden Erfindung eine 12α-HSDH (insbesondere ein NADP⁺-abhängiges Enzym) in einer Form bereitzustellen, die für den präparativen Einsatz bei der pharmazeutischen Wirkstoffsynthese in technischem Maßstab, wie z.B. bei der enzymkatalysierten Oxidation von Cholsäure zu 12-Ketochenodeoxycholsäure (12-Keto-CDCS), geeignet ist.

### Figurenbeschreibung

### In den beiliegenden Figuren zeigt

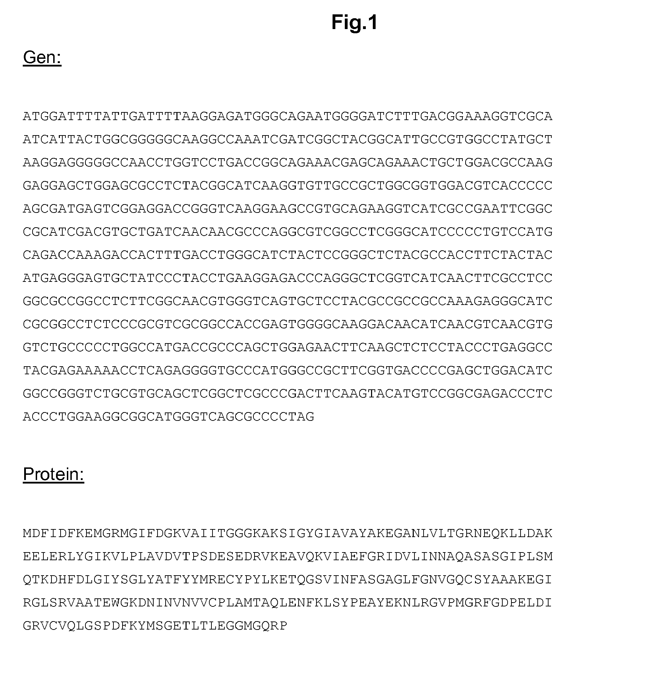

Figur 1 die Gen- bzw. die Proteinsequenz der erfindungsgemäßen Lang-Version von 12α-HSDH (HSDH_lang).

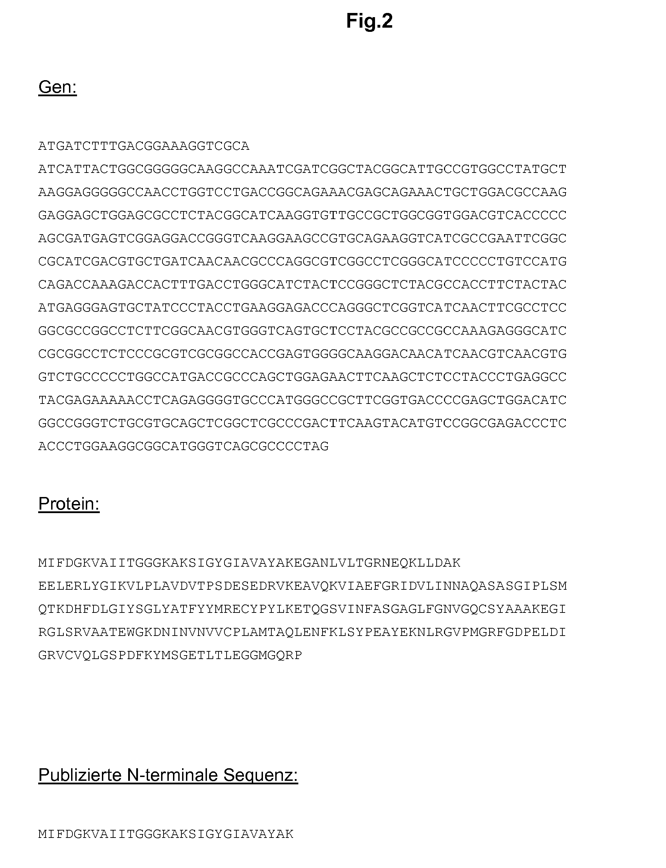

Figur 2 die Gen- bzw. die Proteinsequenz der erfindungsgemäßen Kurzversion der 12α-HSDH (HSDH_kurz) sowie im Vergleich dazu die publizierte unvollständige Teilsequenz gemäß Braun, M. et al., a.a.o.

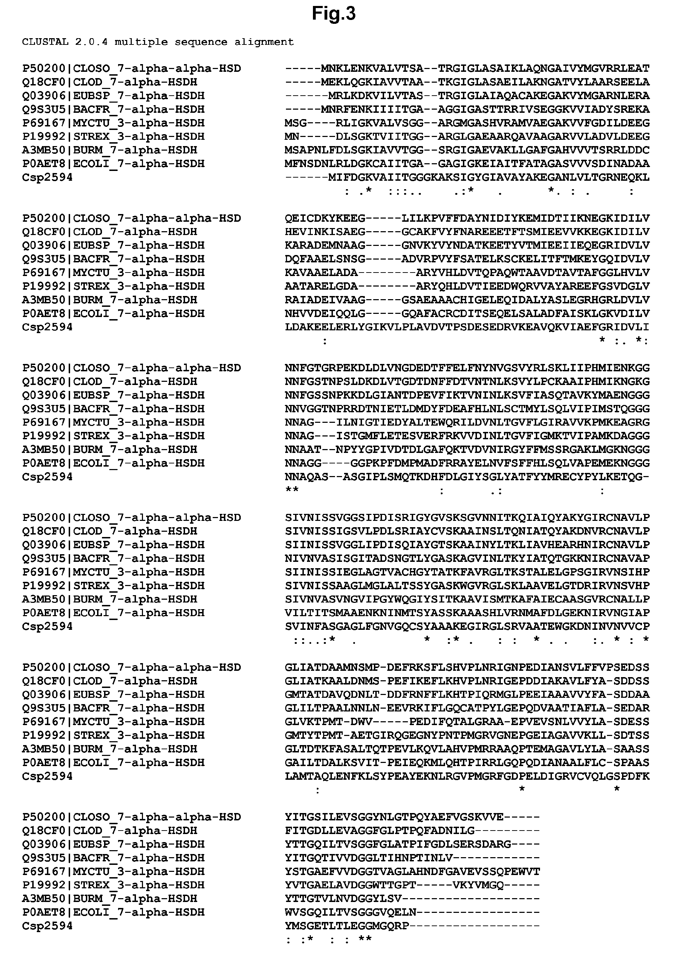

Figur 3 den Ausschnitt eines Multisequenzalignments bekannter mikrobieller HSDH und der erfindungsgemäßen HSDH. Die hochkonservierten Positionen sind mit "*", die variablen mit ":" markiert. Es wurden mikrobielle Hydroxysteroiddehydrogenasen (linke Spalte zeigt die zugehörige *Accession* Nummer und den Herkunftsorganismus), deren Funktion auf Proteinebene untersucht wurde oder die Orthologe in nahe verwandten Arten besitzen, mit der erfindungsgemäß ermittelten Sequenz HSDH_kurz (Csp2594) verglichen. Die bekannten HSDH stammen aus *Escherichia coli* (ECOLI), *Burkholderia mallei* (BURM), *Bacteroides fragilis* (BACFR), *Clostridium sordellii*(CLOSO), *Clostridium difficile* (CLOD), *Eubacterium sp.* (EUBSP), *Mycobacterium tuberculosis* (MYCTU), *Streptomyces exfoliatus* (STREX).
Figur 4 die Expression von erfindungemäßen 12α-HSDH Enzymen in Zelllysaten von BL21- und Rosetta™ (DE3)-Zellen nach 4 bzw. 22 h. In BL21- und Rosetta™ (DE3)-Zellen wurden 12α-HSDH ("lang" und "kurz") entweder 4 oder 22 h exprimiert. Anschließend wurden die Zellen aufgeschlossen und 20 µg Protein aufgetragen. Das Zielprotein (12α-HSDH) ist bei 27 kDa zu finden.
Figur 5 den Verlauf der Absorption bei 340 nm während der Darstellung von 12K-CDCS im 500 ml-Maßstab.
Figur 6 ein Dünnschichtchromatogramm von Reaktionsansätzen zur Bestätigung der Regioselektivität der 12α-HSDH, wobei als Referenz Cholsäure (1) und 12-Ketochenodesoxycholsäure (2) dienten; damit wurden die Reaktionsansätze mit HSDH_lang (3) und HSDH_kurz (4) verglichen.

### Kurzfassung der Erfindung

Obige Aufgabe wurde überraschenderweise durch erstmalige Aufklärung der kodierenden Nukleinsäuresequenz und Beschreibung der korrekten und vollständigen Aminosäuresequenz des in *Clostridium* Gruppe P, strain C48-50 vorkommenden 12α-HSDH-Enzyms gelöst.

Überraschenderweise wurde insbesondere festgestellt, dass die in der Literatur beschriebene N-terminale Aminosäuresequenz nicht der tatsächlichen N-terminalen Aminosäuresequenz entspricht, und dass zusätzlich die 12α-HSDH in einer Lang-Version (HSDH_lang) und in einer N-terminal verkürzten Kurzversion (HSDH_kurz) existiert.

Die erfindungsgemäße Lösung obiger Aufgabe erscheint um so überraschender, als im Stand der Technik nicht erkannt worden war, dass 12α-HSDH-Enzyme unterschiedlicher Länge, insbesondere mit unterschiedlichem N-Terminus, existieren und die fehlerhafte Sequenzinformation aus dem Stand der Technik eine korrekte Primer-Synthese und damit eine Lokalisierung und Amplifizierung der kodierenden Sequenz verhinderte.

Darüber hinaus wurden im Nachgang zu den Arbeiten von Braun, M. et al. a.a.O. systematischere Untersuchungen mit publizierten Sequenzen für Enzyme durchgeführt, die zur Klasse der kurzkettigen Dehydrogenasen/Reduktasen gehören, wozu unter anderem auch die 12α-HSDH gehört. Für diese Gruppe von Enzymen wurde von Oppermann et al. in Chemo-Biological Interactions, 2003, **143 - 144,** 247-253 ein charakteristisches N-terminales Sequenzmotiv, nämlich T-G-X₃-G-X-G postuliert. Dieses Sequenzmotiv ist in der publizierten 12α-HSDH-Sequenz aus dem Jahre 1991 (Braun, M. et al. a.a.o.) nicht zu finden, wodurch die Glaubwürdigkeit der ursprünglich offenbarten partiellen Sequenzinformation für 12α-HSDH in den Augen des Fachmanns bis dato in Frage gestellt war.

### Detaillierte Beschreibung der Erfindung

### 1. Bevorzugte Ausführungsformen

Ein erster Gegenstand der Erfindung betrifft 12α-Hydroxysteroiddehydrogenasen (12α-HSDHs) erhältlich aus *Clostridium* sp. mit einem Molekulargewicht, bestimmt durch SDS-Polyacrylamid-Gelelektrophorese (SDS-PAGE) unter reduzierenden Bedingungen im Bereich von mehr als etwa 26 kD, insbesondere mehr als etwa 26, 5, wie etwa 27 bis 30 kD, und einem berechneten Molekulargewicht von mehr als etwa 29 kD, insbesondere etwa 29,1 bis 29, 5 kD, wie insbesondere 29,359 kD für HSDH_lang bzwetwa 27,8 für (HSDH_kurz). Die Molekulargewichtangaben beziehen sich dabei auf das Molekulargewicht der Proteinuntereinheiten des Enzyms; ohne darauf beschränkt zu sein, besteht das native Protein z.B. aus 4, insbesondere in etwa gleich großen, solchen Untereinheiten.

Insbesondere ist ein derartiges Protein erhältlich aus *Clostridium* sp. group P strain 48-50 (DSM4029), und kann z.B. in einer spezifischen Aktivität im Bereich von mehr als etwa 10 U/mg, wie z.B. 20 bis 100 U/mg oder 25 bis 80 U/mg bereitgestellt werden. Die Bestimmung der spezifischen Aktivität erfolgt dabei unter den im experimentellen Teil angegebenen Standardbedingungen.

Gegenstand der Erfindung sind insbesondere 12α-HSDHs, umfassend wenigstens eines der folgenden Aminosäure-Sequenzmotive:
a) LINN (SEQ ID NO:5)
b) RMGIFD (SEQ ID NO: 11)
c) N-terminale Sequenz, ausgewählt unter
   (1) MDFIDFKEMGRMGIFDGKVAIITGGGKAKSIGYGIAVAYAK (SEQ ID NO: 6)
   (2) MDFIDFKEMGRMGI (SEQ ID NO:7)
   (3) ITGGGKAKSIGYGIA (SEQ ID NO:8)
   (4) IFDGK (SEQ ID NO: 9)
   (5) GIFDGK (SEQ ID NO: 10)
d) FGDPELDI (SEQ ID NO:13) oder davon abgeleitete Sequenzen, wie z.B.: GDPELDI, FGDPELD, DPELDI, FGDPEL, GDPEL, DPELD, GDPELD Weiterhin sind die erfindungsgemäßen Enzyme dadurch gekennzeichnet, dass sie kein N-terminales, (d.h. im Bereich des N-terminalen Endes von etwa 1 bis 30 Aminosäureresten) Sequenzmotiv des Typs TGX₃GXG, worin X für beliebige Aminosäurereste steht, aufweisen.

Gegenstand der Erfindung sind insbesondere 12α-HSDHs,
a) umfassend eine der Aminosäuresequenzen gemäß SEQ ID NO: 2 oder 4, jeweils beginnend bei Position +1 oder +2; oder
b) umfassend eine von einer Sequenz gemäß a) abgeleiteten Aminosäuresequenz mit einer prozentualen Sequenz-Identität von wenigsten 60 %; oder
c) kodiert von einer ein Protein gemäß a) und b) kodierenden Nukleinsäuresequenz; oder
d) kodiert von einer kodierenden Nukleinsäuresequenz gemäß SEQ ID NO: 1 oder 3; oder von einer davon abgeleiteten, an die jeweilige Codon-Nutzung eines zur Expression verwendeten Organismus angepassten Sequenz; oder
e) kodiert von einer der Nukleinsäuresequenzen gemäß SEQ ID NO: 1 oder 3 abgeleiteten kodierenden Sequenz mit einer prozentualen Sequenz-Identität von wenigstens 60 %.

Die Anpassung der Nukleinsäuresequenz an die Conon-Nutzung kann dabei nach üblichen Methoden erfolgen, wie z.B. unter zugänglich unter: http://slam.bs.jhmi.edu/cgibin/gd/gdRevTrans.cgi.

Ein weiterer Gegenstand der Erfindung betrifft 12α-HSDH-Mutanten mit modifizierter Cosubstrat-Nutzung und insbesondere solche Mutanten, abgeleitet von einer 12α-Hydroxysteroiddehydrogenase gemäß obiger Definition, mit wenigstens einer die Cosubstrat-Nutzung modifizierenden Mutation im Sequenzmotiv VLTGRNE (SEQ ID NO: 12). Nicht-limitierende Beispiele für solche Mutanten umfassen solche mit wenigstens eine der folgenden Aminosäuresubstitutionen in SEQ ID NO:12: G →D ; R →A.

Gegenstand der Erfindung sind insbesondere 12α-HSDHs nach obiger Definition, erhältlich durch heterologe Expression wenigstens einer der oben beschriebenen12α-HSDH-kodierenden Nukleinsäuresequenzen, insbesondere solche rekombinant hergestellten Enzyme, exprimiert in einem nicht-pathogenen Mikroorganismus, wie z.B. exprimiert in einem Bakterium der Gattung Escherichia, insbesondere der Spezies E. coli.

Gegenstand der Erfindung sind zudem Nukleinsäuresequenzen gemäß obiger Definition; Expressionskassetten, umfassend wenigstens eine solche kodierende Nukleinsäuresequenz unter der genetischen Kontrolle wenigstens einer regulativen Nukleinsäuresequenz; Vektoren, umfassend wenigstens eine solche Expressionskassette; sowie nach rekombinanter Mikroorganismen, welche wenigstens eine solche Nukleinsäuresequenz oder Expressionskassette tragen oder mit wenigstens einem solchen Vektor transformiert ist.

Außerdem betrifft die Erfindung ein Verfahren zur Herstellung einer 12α-HSDH gemäß obiger Definition, wobei man einen erfindungsgemäßen rekombinanten Mikroorganismus kultiviert und die exprimierte 12α-HSDH aus der Kultur isoliert.

Ein weiterer Gegenstand der Erfindung ist ein Verfahren zur enzymatischen Oxidation von 12α-Hydroxysteroiden, wobei man das Hydroxysteroid in Gegenwart einer erfindungsgemäßen 12α-HSDH umsetzt, und wenigstens ein gebildetes Oxidationsprodukt aus dem Reaktionsansatz gegebenenfalls isoliert. Die Reaktion kann dabei aerob (d.h. in Gegenwart von Sauerstoff) oder anaerob (d.h. im wesentlichen unter Sauerstoffausschluss), insbesondere aerob durchgeführt werden.

Insbesondere kann dabei das Hydroxysteroid Cholsäure (CS) oder eine Cholsäurederivat, wie insbesondere ein Salz, Amid oder Alkylester, ist. Vorzugsweise wird dabei CS oder ein Derivat davon zu 12-Ketochenodesoxycholsäure (12-Keto-CDCS) oder zum entsprechenden Derivat umgesetzt. Insbesondere erfolgt dabei die Reaktion in Gegenwart und unter stöchiometrischem Verbrauch von NADP⁺ oder NAD⁺ erfolgt.

Ein weiterer Gegenstand der Erfindung ist ein Verfahren zur enzymatischen Reduktion von 12-Ketosteroiden, wobei man das Ketosteroid in Gegenwart einer erfindungsgemäßen 12α-HSDH umsetzt und ein gebildetes Reduktionsprodukt aus dem Reaktionsansatz gegebenenfalls isoliert. Die Reaktion kann dabei aerob oder anaerob, insbesondere aerob durchgeführt werden.

Dabei ist das Ketosteroid insbesondere 12-Keto-CDCS oder ein Derivat davon, wie insbesondere ein Salz, Amid oder Alkylester. Dabei wird das Ketosteroid oder dessen Derivat zum korrespondierenden 12α-Hydroxysteroid oder dessen Derivat reduziert. Insbesondere erfolgt dabei die Reaktion in Gegenwart von NADPH oder NADH.

In einer bevorzugten Ausführungsform obiger Redox-Reaktionen können die verbrauchten Redox-Äquivalente elektrochemisch oder enzymatisch regeneriert werden. Geeignete enzymatische Regenerationssysteme sind eingangs bereits beschrieben worden. Auf die Offenbarung dieser Druckschriften wir ausdrücklich Bezug genommen. Nichtlimitierende Beispiele dafür geeigneter Enzyme sind Glutamatdehydrogenase, Alkoholdehydrogenase und Lactatdehydrogenase. Elektrochemische Regenerationsverfahren basieren z.B. auf Hydridorhodium-Redoxkatalysatoren, wie z.B. beschrieben in der WO-A-01/88172, worauf hiermit Bezug genommen wird.

In einer weiteren Ausgestaltung obiger Redoxreaktionen, können diese mit einer 12α-HSDH in immobilisierter Form erfolgen. Gegebenenfalls verwendete Enzyme für die Kofaktorregeneration können ebenfalls immobilisiert sein

Ein weiterer Gegenstand der Erfindung ist ein derartiger Bioreaktoren zur Durchführung obiger Redoxreaktionen, oder von solchen Teilreaktionsschritte im Rahmen eines synthetischen Gesamtprozesses.

Ein weiterer Gegenstand der Erfindung betrifft ein Verfahren zum qualitativen oder quantitativen Nachweis von 12-Ketosteroiden bzw. 12α-Hydroxysteroiden, wobei man das Steroid einer durch eine erfindungsgemäße 12α-HSDH katalysierten Redoxreaktion in Gegenwart von Redoxäquivalenten durchführt, eine Änderung in der Konzentration der Redoxäquivalente bestimmt und daraus den Gehalt an 12-Ketosteroiden bzw. 12α-Hydroxysteroiden qualitativ oder quantitativ ermittelt.

Gegenstand der Erfindung sind weiterhin Verfahren zur Synthese von Ursodesoxycholsäure (UDCS) aus Cholsäure (CS), umfassend wenigstens einen von einer erfindungsgemäßen 12α-HSDH katalysierten Reaktionsschritt. Dieser Reaktionsschritt kann dabei aerob oder anaerob, insbesondere aerob durchgeführt werden. Drei geeignete Reaktionssequenzen sind z.B. von Sutherland et al., a.a.O., beschrieben worden, worauf hiermit Bezug genommen wird. Folgende Synthesewege 1 bis 3 (wobei UCS für Ursocholsäure steht und CDCS für Chenodesoxycholsäure steht) wurden beschrieben:

### 1. Weg

- CS → 12-Keto-CDCS: (enzymatisch durch 12α-HSDH)
- 12-Keto-CDCS → 12-Keto-UDCS: (enzymatisch durch 7α und 7β-HSDH)
- 12-Keto-UDCS → UDCS: (chemisch Wolfif-Kishner Reduktion)

### 2. Weg

- CS → UCS: (enzymatisch durch 7α und 7β-HSDH)
- UCS → 12-Keto-UDCS: (enzymatisch durch 12α-HSDH)
- 12-Keto-UDCS → UDCS: (chemisch Wolfif-Kishner Reduktion)

### 3. Weg

- CS → 12-Keto-CDCS: (enzymatisch durch 12α-HSDH)
- 12-Keto-CDCS → CDCS: (chemisch Wolfif-Kishner Reduktion)
- CDCS → UDCS: (ganze *Clostridium absonum* Zellen)

Gegenstand der Erfindung ist aber insbesondere folgender, 4. Weg:

### 4. Weg

Dieser betrifft die Herstellung einer Ursodesoxycholsäure der Formel (1) 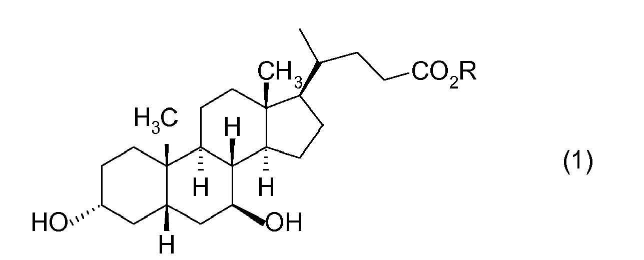 worin
R für Alkyl, NR¹R², H, ein Alkalimetallion oder N(R³)₄⁺steht, worin die Reste R³ gleich oder verschieden sind und für H oder Alkyl stehen,
wobei man
a) eine Cholsäure (CS) der Formel (2) 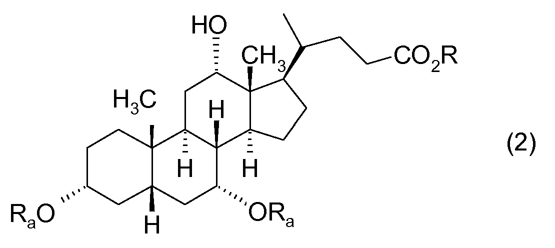 worin R die oben angegebenen Bedeutungen besitzen, und die Reste Rₐ gleich oder verschieden sind und für H oder Acyl stehen, in Gegenwart einer erfindungsgemäßen12α-HSDH zur korrespondierenden 12-Ketochenodesoxycholsäure (12-Keto CDCS) der Formel (3) 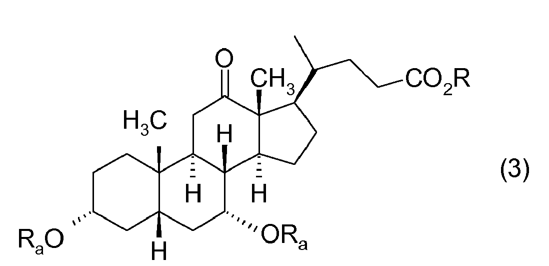 worin R und Rₐ die oben angebebenen Bedeutungen besitzen, oxidiert und anschließend
b) 12-Keto-CDCS der Formel (3) durch Desoxygenierung, wie z.B. durch Wolfif-Kishner Reduktion zu Chenodesoxycholsäure (CDCS) der Formel (4) 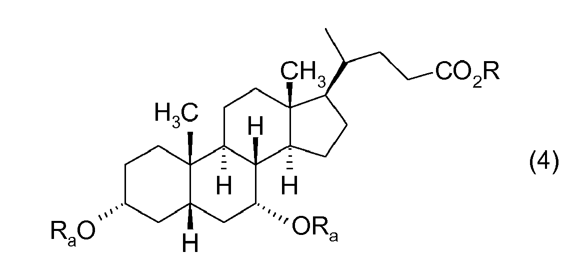 worin R und Rₐ die oben angebebenen Bedeutungen besitzen, umsetzt und
c) CDCS der Formel (4) in Position 7 chemisch oxidiert zur 7-Keto-Lithocholsäure (KLCS) der Formel (5) 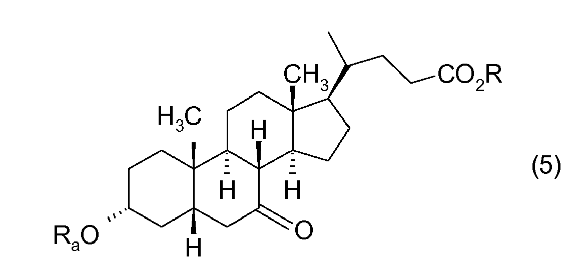 worin R und Rₐ die oben angebebenen Bedeutungen besitzen; und
d) KLCS der Formel (5) reduziert und, wenn Rₐ für Acyl steht, diese Acylgruppe gegebenenfalls abspaltet, und
e) das Reaktionsprodukt gegebenenfalls weiter aufreinigt.

Dabei kann, wenn Rₐ für Acyl steht, diese Acylgruppe nach Durchführung der Reaktionsstufe b) oder d) gegebenenfalls abspalten werden.

Weiterhin kann die Reaktion von Stufe a) insbesondere in Gegenwart von NAD(P)⁺erfolgen.

Weiterhin kann verbrauchtes NAD(P)⁺ in an sich bekannter Weise elektrochemisch oder enzymatisch regeneriert werden und /oder die verwendeten Enzyme können in immobilisierter Form erfolgt.

### 2. Allgemeine Definitionen

Werden keine anderen Angaben gemacht, so bezeichnet der Begriff "12 α-HSDH" ein Dehydrogenaseenzym, welches wenigstens die stereospezifische Oxidation von Cholsäure zu 12-Keto-Chenodesoxycholsäure unter stöchiometrischem Verbrauch von NAD⁺ bzw. NADP⁺ katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen.

Unter einer "Reinform" versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt. Die spezifische Aktivität eines erfindungsgemäßen 12α-HSDH-Enzyms liegt dabei in dem oben angegebenen Bereich.

Unter einem "Redoxäquivalent" versteht man eine als Elektronendonor bzw. Elektronenakzeptor brauchbare, niedermolekulare organische Verbindung, wie beispielsweise Nikotinamidderivate wie NAD⁺ und NADH⁺ bzw. deren reduzierte Formen NADH bzw. NADPH.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das NH₄⁺-Salz bzw. solche Ammoniumsalze umfasst, worin wenigstens ein Wasserstoffatom durch einen C₁-C₆-Alkylrest ersetzt sein kann. Typische Alkylreste sind insbesondere C₁-C₄-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäßer Verbindungen sind insbesondere Niedrigalkyl-Ester, wie z.B. C₁-C₆-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono- oder Di-C₁-C₆-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und C₁-C₆-Alkylgruppen, wie z.B. Benzoyl oder Toluoyl.

Die erfindungsgemäß eingesetzten bzw. hergestellten Hydroxysteroidverbindungen, wie Cholsäure, Glykocholsäure, Taurocholsäure Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Unter einer "Immobilisierung" versteht man erfindungsgemäß die kovalente oder nichtkovalente Bindung eines erfindungsgemäß verwendeten Biokatalysators, wie z.B. einer 12α-HSDH an einem festen, d.h. in dem umgebenden flüssigen Medium im Wesentlichen unlöslichen, Trägermaterial.

### 3. Weitere Ausgestaltungen der Erfindung

### 3.1 Proteine

Die vorliegende Erfindung ist nicht auf die konkret offenbarten Proteine bzw. Enzyme mit 12α-HSDH-Aktivität beschränkt, sondern erstreckt sich vielmehr auch auffunktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 12α-HSDH-Aktivität, besitzen.

So versteht man beispielsweise unter "funktionalen Äquivalenten" Enzyme, die im verwendeten Test auf 12α-HSDH-Aktivität eine um mindestens 1 % , wie z.B. mindestens 10% oder 20 %, wie z.B. mindestens 50 % oder 75% oder 90 % höhere oder niedrigere Aktivität eines Enzyms, umfassend eine hierin definierte Aminosäuresequenz aufweisen. Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 12α-HSDH -Aktivität kann mit Hilfe verschiedener bekannter Tests nachgewiesen werden. Ohne darauf beschränkt zu sein, sei ein Test unter Verwendung eines Referenzsubstrates, wie z. B. Cholsäure, unter standardisierten Bedingungen wie im experimentellen Teil definiert, zu nennen.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | Beispiele der Substitution |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Ankeroder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; lke et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3.2 Nukleinsäuren und Konstrukte

### 3.2.1 Nukleinsäuren

Gegenstand der Erfindung sind auch Nukleinsäuresequenzen, die für ein Enzym mit 12α-HSDH-Aktivität kodieren.

Die vorliegende Erfindung betrifft auch Nukleinsäuren mit einem bestimmten Idetitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameters: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| Pairwise alignment parameter: | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse:
http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischerStandard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1 oder 3 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO: 1 oder 3 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO: 1 und 3, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO: 7 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 7 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise
- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Nicht limitierende Beispiele solcher hot spot-Regionen der erfindingsgemäßen HSDH sind im Folgenden zusammengefasst:

35-40, insbesondere 37-38, (jeweils bezogen auf die Aminosäuresequenz von HSDH_kurz (SEQ ID NO: 4).

### 3.2.2 Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO: 1 oder 3 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, lacI^{q-}, T7-, T5-, T3-, gal-, trc-, ara-, rhaP (rhaP_{BAD})SP6-, lambda-P_{R}- oder im lambda-P_{L}-Promotor enthalten, die vorteilhafterweise in gramnegativen Bakterien Anwendung finden. Weitere vorteilhafte Regulationssequenzen sind beispielsweise in den gram-positiven Promotoren amy und SP02, in den Hefe- oder Pilzpromotoren ADC1, MFalpha, AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in Streptomyces pIJ101, plJ364, plJ702 oder plJ361, in Bacillus pUB110, pC194 oder pBD214, in Corynebacterium pSA77 oder pAJ667, in Pilzen pALS1, plL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 3.3 Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 12α-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 3.4 Herstellung von UDCS

### 3.4.1 Einleitung

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: β -Konfiguration, CDCS: α-Konfiguration). Zur Herstellung von kommerziellen Mengen UDCS wird bisher bevorzugt ein Verfahren verwendet, bei dem CDCS als Rohstoff eingesetzt wird. CDCS wiederum wird vorzugsweise aus Cholsäure (CS) hergestellt.

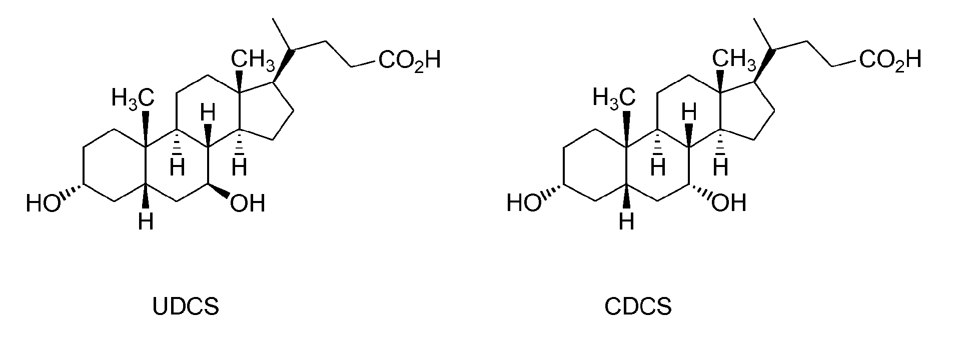

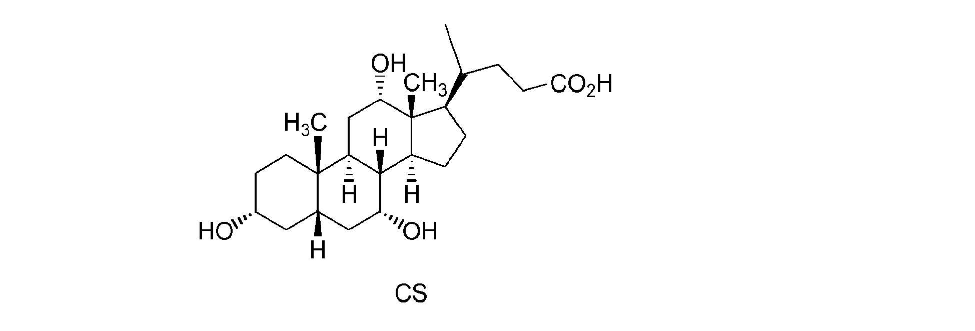

### 3.4.2 Herstellung von CDCS

Als Rohstoff für die Herstellung von CDCS (CAS 474-25-9) wird CS (CAS 81-25-4) verwendet. Die klassische chemische Route 1 bedient sich ausschließlich chemischen Verfahrensschritten. In diesem Fall sind vier Schritte erforderlich, um CS zu CDCS umzuwandeln. Die alternative Route 2 umfasst die Enzym-katalysierte Umsetzung. Dieser Weg führt in nur zwei Schritten von CS zu CDCS.

### 3.4.2.1 Route 1 (chemischer Weg)

Im ersten Schritt dieser Synthese wird die Carbonsäuregruppe der CS zum Methylester (CDCS I, CAS 1448-36-8) verestert. Es schließt sich die regioselektiv verlaufende Acetylierung der Hydroxygruppen in den Positionen 3 und 7 an. Das Acetylierungsprodukt, 3,7-Di-O-acetylcholsäuremethylester (CDCS II, CAS 3749-87-9) fällt kristallin an und wird isoliert. In der Folgestufe (Schritt 3) wird die freie Hydroxygruppe in Position 12 osidert. Der 3,7-Di-O-acetyl-12-ketochlansäuremethylester (CDCS III, CAS 4651-67-6) wird im vierten und letzten Schritt in einer Wolfif-Kishner-Reduktion zu CDCS desoxygeniert.

### 1. Schritt: Veresterung

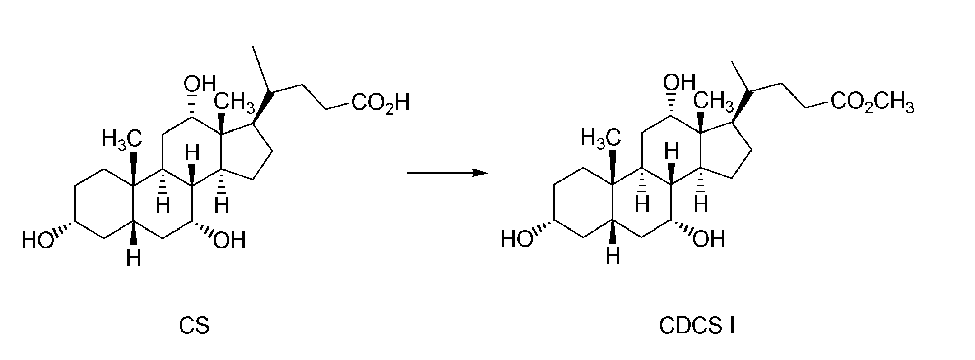

### 2. Schritt: Acetylierung

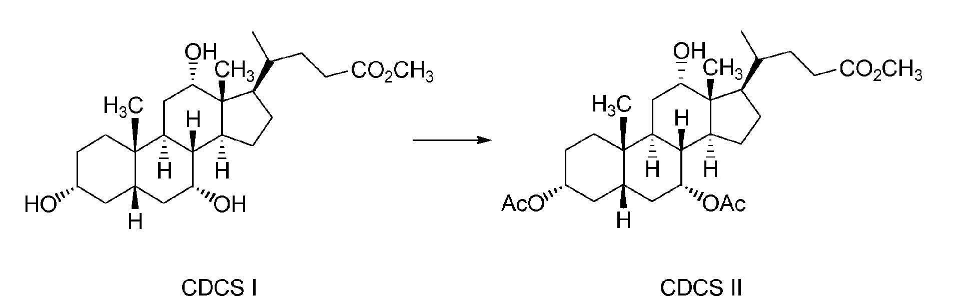

### 3. Schritt: Oxidation

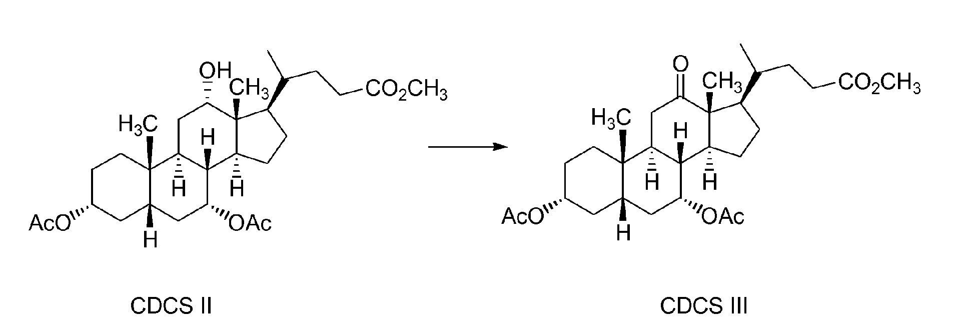

### 4. Schritt: Desoxygenierung

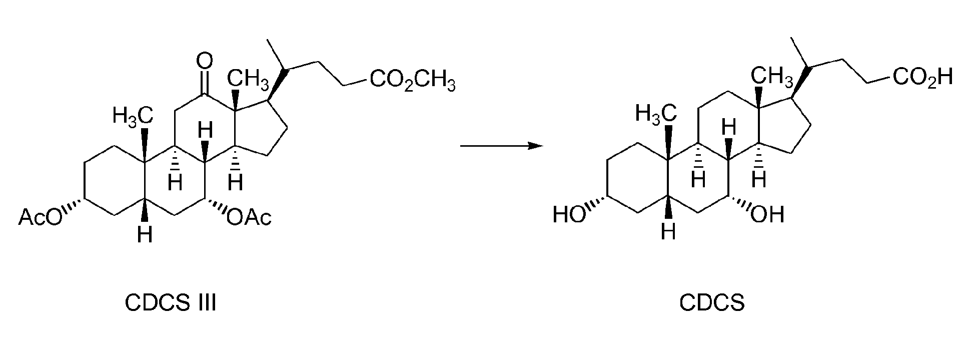

### Im Einzelnen wird der Prozess wie folgt durchgeführt:

In Stufe 1 wird CS mit Methanol säurekatalysiert zum Cholsäuremethylester (CDCS I) verestert. Es folgt die regioselektive Acetylierung der Hydroxylgruppen in den Positionen 3 und 7 mit Acetanhydrid. Zur Reaktion wird eine organische Stickstoffbase und optinoal ein Acylierungskatalysator verwendet. Durch Optimierung der Reaktionszeit wird hier ein Maximum der Diacetylverbindung (CDCS II) erreicht. Das Produkt wird nach Kristallisation isoliert und getrocknet. Acetylierungsbedingungen, insbesondere die Kombination Acetanhydrid, Triethylamin und DMAP werden in der EP 0 424 232 beschrieben. Die Selektivität der Acetylierung entscheidet über die spätere Qualität des (Zwischen-) Produkts CDCS. Das Nebenprodukt 3-O-Monoacetylcholsäuremethylester führt im weiteren Syntheseverlauf zu Lithocholsäure. Diese ist toxisch und in den Monographien des Endprodukts UDCS auf einen niedrigen Wert limitiert (Ph. Eur. 0,1 %, USP 0,05 %). Bei einer Überacetylierung zum 3,7,12-Tri-O-acetylcholsäuremethylester enthält die später erhaltene CDCS anteilig mehr CS als Verunreinigung.

Die Oxidation der CDCS II zu CDCS III erfolgt mit wässriger Natriumhypochloritlösung. Das Produkt fällt aus der Reaktionslösung aus, wird abfiltriert und getrocknet. Auch dieses Procedere wird in der EP 0 424 232 beschrieben. Generell finden sich in der Literatur noch andere Oxidationsmittel als Alternativen, wie Chromsäure.

Zur Desoxygenierung der CDCS III zu CDCS sind verschiedene Varianten der Wolff-Kishner-Reduktion bekannt. Bei einer Methode wird CDCS III mit Hydrazin und Natriumhydroxid in Triethylenglycol bei 200°C umgesetzt. Das Produkt wird durch Ansäuern mit Salzsäure aus der Reaktionslösung gefällt, anschließend abfiltriert und getrocknet. Eine andere Methode ist in EP 0 424 232 beschrieben und arbeitet bei niedrigerer Temperatur. CDCS III wird hier mit Hydrazin und Kaliumhydroxid in 2-Butanol umgesetzt. Das Produkt wird aus Wasser wie bei Variante 1 durch Zugabe von Salzsäure gefällt.

Die nach diesem Verfahren erhaltene CDCS besitzt eine definierte und spezifizierte Qualität, die geeignet ist, um nach dem später beschriebenen Verfahren UDCS in Arzneibuchqualität herzustellen.

### 3.4.2.2 Route 2 (enzymatischer Weg)

Als Alternative zum ausschließlich chemischen Verfahren wird erfindungsgemäß eine enzymkatalysierte Oxidation von CS zu 12-Ketochenodesoxycholsäure (12-Keto-CDCS, CAS 2458-08-4), die dann weiter zu CDCS umgesetzt wird, bereitgestellt. Dieser Syntheseweg beinhaltet nur zwei Schritte und ist damit im Vergleich zu der rein chemischen Route deutlich einfacher durchzuführen.

### 1. Schritt: enzymatische Oxidation

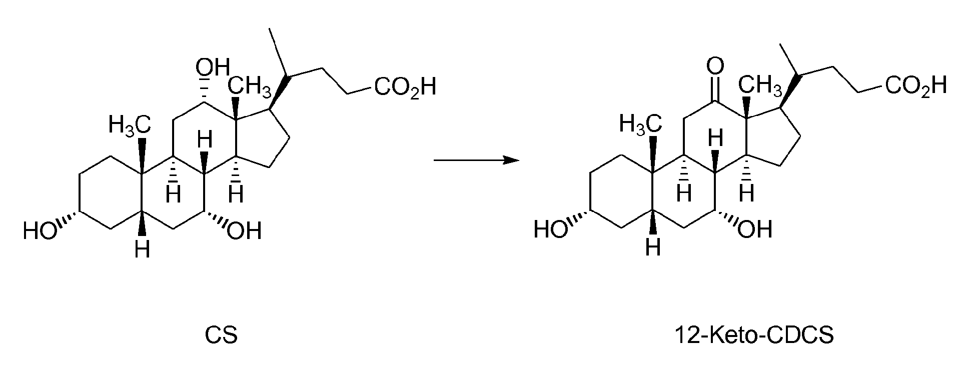

### 2. Schritt: Desoxygenierung

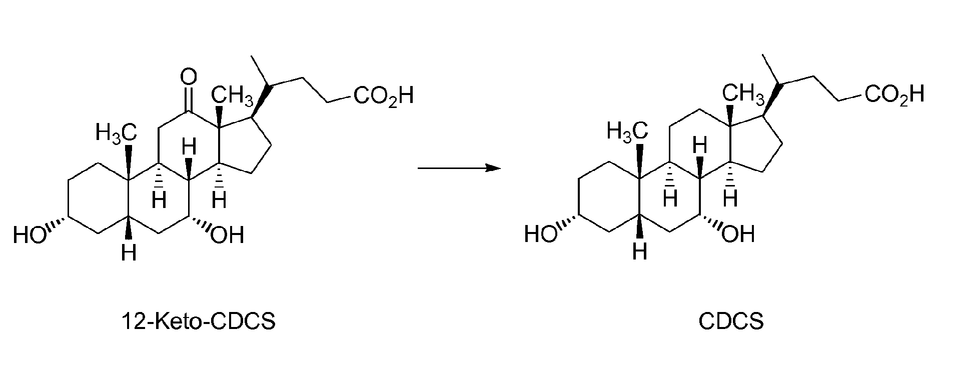

Gemäß Schritt 1 wird Cholsäure mittels 12α-HSDH zu 12-Ketochenodesoxycholsäure (12-Keto-CDCS) NADP⁺-abhängig oxidiert. Diese Reaktion ist reversibel. 12α-HSDHs gehören der Enzymklasse 1.1.1.176 an und werden hauptsächlich in Bakterien der Gattung *Clostridium* gefunden. Es existieren sowohl NADP⁺-abhängige (Harris and Hylemon (1978) Biochim Biophys Acta 528(1): 148-57), als auch NAD⁺-abhängige Vertreter (Macdonald et al. 1976) Biochim Biophys Acta 450(2): 142-53.

Der einzige bekannte Mikroorganismus, der eine hohe 12α-HSDH-Aktivität in Abwesenheit anderer HSDH exprimiert, stellt *Clostridium sp.* group P strain 48-50 DSM 4029 dar (Macdonald *et al.* 1979. a.a. O.). Deshalb wurde dieser Organismus bisher als Produzent von 12α-HSDH eingesetzt, wobei eine anspruchsvolle, anaerobe Fermentation mit kostenintensivem Medium nötig ist (Macdonald 1981) Experientia 37(5): 451-2. Allerdings konnte letzteres durch Hefeautolysat ersetzt werden (Braun, M. *et al.* 1991, a.a.O).

Die enzymatische Oxidation erfolgt erfindungsgemäß bevorzugt mittels einer erfindungsgemäßen 12α-HSDH (Lang- oder Kurzversion) und Kofaktorregenerierung mittels einer ADH , wie z.B. ADH ms oder ADH t.

Die Desoxygenierung gemäß Schritt 2 ist eine klassische chemische Wolff-Kishner-Reduktion und wird analog zur oben beschriebenen Desoxygenierung der CDCS III durchgeführt. Ein wesentlicher Vorteil dieser Route ist, dass durch die Selektivität des Enzyms die Verunreinigung Lithocholsäure nicht entsteht.

### 3.4.3.3 Herstellung von UDCS

Als Rohstoff für UDCS (CAS 128-13-2) wird CDCS verwendet. Im ersten Syntheseschritt wird die Hydroxylgruppe in Position 7 der CDCS zum entsprechenden Keton oxidiert. Es resultiert die 7-Ketolithocholsäure (3α-Hydroxy-7-ketocholansäure, kurz: KLCS, CAS 4651-67-6). Im zweiten Schritt folgt die stereoselektive Reduktion der Ketogruppe in Position 7. Ziel ist es, mit möglichst hoher Diastereoselektivität UDCS zu erhalten. In der Regel enthält die UDCS direkt nach der Reduktion noch einige Prozent des Diastereomers CDCS. Um zum Wirkstoff UDCS zu gelangen, muss UDCS roh in einem dritten Schritt gereinigt werden.

### 1. Schritt: Oxidation

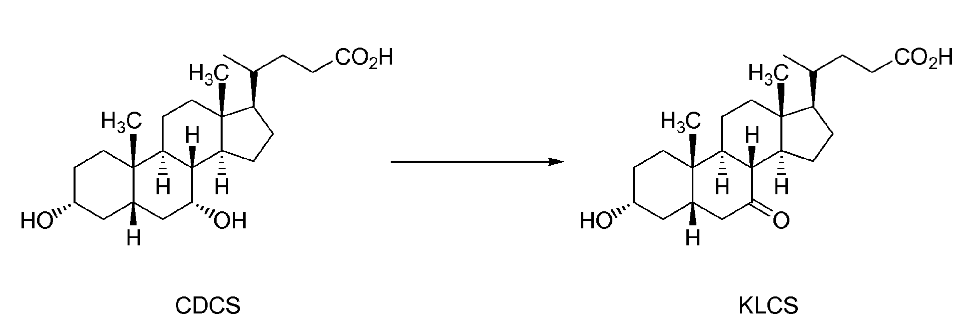

### 2. Schritt: Reduktion

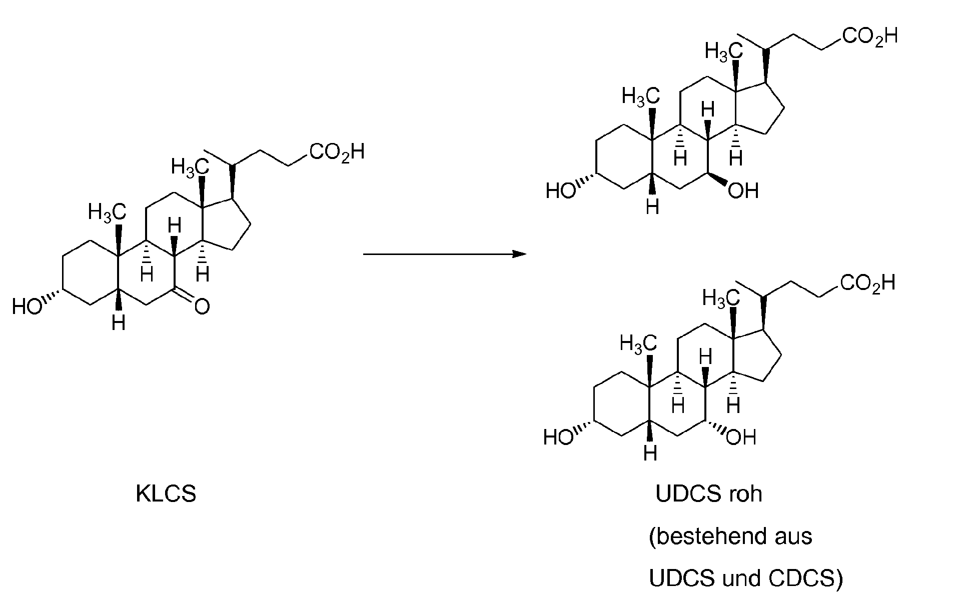

### 3. Schritt: Reinigung

### UDCS roh -> UDCS rein

Die Oxidation der CDCS erfolgt üblicherweise mit wässriger Natriumhypochloritlösung. In der Literatur findet sich noch die Chromsäureoxidation als Alternative. KLCS fällt als Feststoff an, der dann im zweiten Schritt weiter verarbeitet wird. Die Reduktion kann mit Natriummetall in Alkoholen durchgeführt werden. Es resultiert ein Rohprodukt mit einer Zusammensetzung von UDCS : CDCS von ca. 85 : 15. In alternativen Verfahren wird KLCS mit Wasserstoff an einem Nickelkatalysator (Raney-Nickel) in Alkoholen (wie z.B. aliphatischen Alkoholen) als Lösungsmittel zusammen mit einer Base, wie Kalium-t-butylat oder Kaliumhydroxid, reduziert (EP-A-0 230 085). Zusätzlich ist noch die Reduktion mit Kalium und Lithium (höhere Selektivität als Natrium, C. Giordano et al. Angew. Chem. 1985, 97, 510) sowie Zink (ES 489661) und elektrochemisch (US 4 547 271) möglich.

Bei der Aufreinigung von UDCS roh zu UDCS rein handelt es sich um eine Trennung diastereomerer Salze. Sie erfolgt durch Herstellung, Isolierung und nachfolgende Spaltung eines geeigneten Salzes von UDCS. In der Literatur werden folgende alternativen Reinigungsmethoden genannt: Herstellung, Umkristallisation und Spaltung eines korrespondierenden UDCS-Esters (EP-A-0386 538), Extraktionen (JP 60006699) und chromatographische Verfahren.(IT 2000MI1177).

### 3.5 Rekombinante Herstellung von 12α-HSDH

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und.

Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 3.6 Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

### EXPERIMENTELLER TEIL

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wurden wie bei Sambrook et al. (1989) a.a.O. beschrieben durchgeführt werden.

### A. Allgemeine Angaben

### Materialien:

Enzyme und Enzympuffer wurden von Fermentas, St. Leon-Rot oder NEB, Frankfurt bezogen.

| LB-Medium: | |
|---|---|
| Bacto-Trypton | 10 g |
| Hefe-Extrakt | 5 g |
| Natriumchlorid | 5 g |
| doppeltdestilliertes Wasser | ad 1000 ml |

| TB-Medium: | |
|---|---|
| Lösung I: | |
| Bacto-Trypton | 12 g |
| Hefe-Extrakt | 24 |
| Glycerin, wasserfrei | 4 ml |
| doppeltdestilliertes Wasser | ad 900 ml |
| Lösung 11: | |
| Kaliumdihydrogenphosphat | 0,17 M |
| Kaliumhydrogenphosphat | 0,72 M |
| doppeltdestilliertes Wasser | ad 100 ml |

Beide Lösungen wurden nach dem Autoklavieren vereinigt.

### Expressionsvektoren

Zur Expression von 12α-HSDH diente der Vektor pET22b(+), Novagen, Darmstadt, welcher eine MCS unter der Kontrolle eines T7-Promotors und -Transkriptionsstarts enthält und einen T7-Terminator besitzt. Die Expression wird mittels Isopropyl-β-D-thiogalactopyranosid (IPTG) induziert.

Dazu wurden 12α-HSDH kodierende Sequenzen PCR-amplifiziert. Die PCR-Produkte erhielt man unter Verwendung der genomischen DNA von *Clostridium sp.* group P strain 48-50 als Template und der später noch genauer beschriebenen Primerpaare. Die PCR-Produkte wurden auf ein Agarose-Gel aufgetragen, aufgetrennt und aus diesem ausgeschnitten. Anschließend wurden sie mit Hilfe von Ndel und BamHI restringiert und mit dem ebenfalls mit Ndel und BamHI geschnittenen pET22b(+)-Vektor ligiert.

### Mikroorganismen

| **Stamm** | **Genotyp** |
|---|---|
| *Clostridium sp.* group P strain 48-50 | |
| *Escherichia coli* BL21 (DE3) | F⁻ ompT gal dcm Ion hsdS_{B}(r_{B}⁻m_{B}⁻) λ(DE3 [lacI lacUV5-T7 gene 1 ind1 sam7 nin5]) |
| *Escherichia coli* Rosetta™ (DE3) | F⁻ompT hsdsB(R_{B}⁻m_{B}⁻) gal dem λ(DE3 [lacI lacUV5-T7 gene 1 ind1 sam7 nin5]) pLysS-RARE (Cam^{R}) |

### Methoden

### 1. Standardbedingungen für 12α-HSDH Aktivitätsbestimmung

Die Aktivität wird wie folgt definiert: 1 U des Enzyms entspricht der Enzymmenge, welche die Umsetzung von 1 µmol/min einer 5 mM Cholsäurelösung in KaliumphosphatPuffer (50 mM, pH 8,0) bei Raumtemperatur (d.h. ca. 20 °C-23 °C) katalysiert.

Zur Aktivitätsbestimmung wurden 790 µl Kaliumphosphatpuffer (50 mM, pH 8,0), 100 µl Cholsäure (50 mM in Kaliumphosphatpuffer (50 mM, pH 8,0)) und 10 µl zu vermessende Enzymlösung in einer Küvette gemischt. Zum Start der Reaktion wurde 100 µl NADP⁺ (2,5 mM) zugegeben und die Zunahme der Absorption bei 340 nm photometrisch bestimmt. Die Steigung über 30 s wurde bei RT ermittelt. Die Bestimmung der Aktivität erfolgte nach dem Lambert-Beerschen-Gesetz.

### 2. Proteinkonzentrationsbestimmung mittels BCA-Assay

Die Proteinkonzentration einer Lösung wurde bestimmt, indem die Absorption von 20 µl Proteinlösung, wie z.B. einer Zelllysat-Verdünnung bzw. eines resuspendierten Zelldebris-Pellets nach Ultraschallaufschluss, in 200 µl BCA-Lösung (Lösung A:Lösung B 50:1) des Analysen-Kits der Firma Bio-Rad, München bei 562 nm gemessen wurde. Dabei bildet der Bicinchoninsäure (BCA) mit einwertigen Kupferionen, die quantitativ aus der Reduktion zweiwertigen Kupferionen durch das Protein entstehen, eine violette Komplexverbindung, dessen Absorption bei 562 nm photometrisch gemessen werden kann. Die Bestimmung der Konzentration erfolgte über eine Rinderserumalbumin (BSA)-Eichgerade.

### B. Vorversuche zur Genisolierung

Ziel aller experimentellen Arbeiten ist es, einen verbesserten Zugang zu dem Enzym 12α-HSDH zu finden. Um dieses Ziel zu erreichen wurde erfindungsgemäß die Sequenz des für 12α-HSDH kodierenden Gens aufgeklärt.
1.1 Zunächst wurde dies durch Polymerasekettenreaktion (PCR) unter Verwendung degenerierter Oligonucleotidprimer versucht. Die eingesetzten Oligonucleotide wurden einerseits basierend auf der publizierten N-terminalen Aminosäuresequenz (vgl. Braun et al., a.a.O.) von 12α-HSDH konstruiert. Um den Degenerationsgrad gering zu halten, wurden die Primer beginnend mit dem N-terminalen Methionin (nur ein Codon) abgeleitet (Primersequenzen nicht gezeigt). Andererseits ergaben datenbank-gestützte Sequenzvergleiche, dass in HSDHs ein konserviertes Aminosäuremotiv "LVNN" vorliegt. Dieser Bereich wurde zur Konstruktion eines reversen Primers benutzt. Zusätzlich wurde ein weiteres, weniger streng konserviertes Sequenzmotiv PE(Q)DIAN zum Design degenerierter Primer benutzt (Primersequenzen nicht gezeigt). Weitere degenerierte Oligonucleotide wurden mittels des frei zugänglichen Programms CODEHOP entworfen (Rose, T.M. et al., Nucleic Acids Research, 1998. 26(7),1628-1635.)
   Unter Benutzung aller Kombinationen der oben angegebenen degenerierter Primerpaare konnte das gesuchte Gen nicht amplifiziert werden.
1.2 In einem Versuch zur Ermittlung weiterer Peptidsequenzfragmente aus 12α-HSDH wurde versucht, das Enzym aus dem Lysat von *Clostridium sp* group P über eine Kombination aus zwei Affiniätschromatographieschritten aufzureinigen. Dieses Verfahren wurde von Braun et al a.a.O. beschrieben, ließ sich allerdings nicht nacharbeiten

### C. Isolierung der kodierenden 12α-HSDH Sequenz und Charakterisierung des 12α-HSDH Enzyms

### Beispiel 1: Sequenzhomologie-Untersuchungen

Um Zugang zur 12α-HSDH-Sequenz zu erhalten wurde das Genom von *Clostridium sp.* group P strain 48 -50 DSM 4029 sequenziert. Die Suche sowohl nach der publizierten N-terminalen Sequenz als auch die Suche nach dem Motiv "LVNN" in allen offenen Leserastern (ORFs) führte nicht zur Sequenz des für 12α-HSDH kodierenden Gens.

Erst durch Sequenzhomologievergleiche konnte ein Gen identifiziert werden, das die publizierte Teilsequenzinformation in abgewandelter Form enthielt. Die Vergleiche erfolgten mit TBLASTX (Tatusova und Madden (1999) FEMS Microbiol Lett 174 (2), 247-250) und unter folgenden Bedingungen:
Open gap: 5
Extension gap: 2
gap x_dropoff: 50
expect: 10.0
word size: 11

Es wurden die Standardbedingungen von http://www.ncbi.nlm.nih.gov/blast/Blast.cgi?PAGE=Translations&PROGRAM=tblastx&B LAST PROGRAMS=tblastx&PAGE TYPE=BlastSearch&SHOW DEFAULTS=on#i verwendet, Parameter sind dort unter "Algorithm parameters" zu finden.

Im gefundenen Gen (SEQ ID NO:3) fehlt das in der publizierten Teilsequenz angegebene N-terminale Methionin; außerdem ist die publizierte Teilsequenz nicht am N-Terminus des Proteins (bioinformatische Vorhersage des Genstarts mit GLIMMER, CBCB, Maryland, USA) zu finden. Auch das konservierte Motiv "LVNN" ist in 12α-HSDH abgewandelt zu "LINN" (SEQ ID NO: 5).

Aus diesen Gründen konnte der ursprünglich verfolgte Ansatz zur Sequenzaufklärung unter Verwendung degenerierter Oligonucleotide nicht erfolgreich verlaufen. Gerade das Methionin wird regelmäßig verwendet um degenerierte Primer abzuleiten, da es nur durch ein einziges Basentriplett kodiert wird. Ebenso war nicht zu erwarten, dass ausgerechnet 12α-HSDH aus *Clostridium sp.* Abweichungen in der konservierten Sequenz "LVNN" zeigt.

Figur 3 zeigt ein partielles Multisequenzalignment zwischen bekannten mikrobiellen und erfindungsgemäße HSDH.

### Beispiel 2: Amplifizierung des 12α-HSDH Gens und Expression von 12α-HSDH

### 1. Amplifizierung

Dazu wurden folgende Primer verwendet:
Forward lang (lange Enzymversion, Ndel-Schnittstelle):
   GGTATTCCATATGGATTTTATTGATTTTAAGGAGATG (SEQ ID NO: 14).
Forward kurz (kurze Enzymversion, Ndel-Schnittstelle)
   GGTATTCCATATGATCTTTGACGGAAAGGTCGC (SEQ ID NO: 15).
Primer revers (BamH1-Schnittstelle)
   CGGGATCCCTAGGGGCGCTGACCC (SEQ ID NO: 16).

Das Zielgen wurde unter Verwendung von *Pfu*-Polymerase durch PCR amplifiziert.

Als Template diente die genomische DNA von *Clostridium sp.* group P strain 48-50 DSM 4029 (29,4 ng/µl), von der 1 µl eingesetzt wurde. ZurAmplifikation wurde 1 µl der *Pfu*-Polymerase verwendet. Als Puffer diente *Pfu*-Puffer (10x mit MgSO₄) (Fermentas, St. Leon-Rot). Es wurde jeweils 1,5 µl *forward-* und revers-Primer (10 µM) eingesetzt, sowie 2 µl Desoxynukleotidtriphosphate (20 µM). Der Ansatz wurde mit RNase-freiem Wasser auf 50 µl eingestellt. Die Reaktion wurde im Thermocycler der Firma Eppendorf durchgeführt. Der PCR-Ansatz wurde zunächst für 5 min bei 95°C gestartet, um die DNA zu denaturieren. Nun folgten bei der Klonierung der unbekannten DNA-Sequenzen 30 Zyklen beginnend mit einer Denaturierung bei 95°C für 30 s. Nachfolgend wurde der Ansatz für 30 s mittels Temperaturgradient auf jeweils 25-45°C abgekühlt, um ein Annealing der degenerierten Primer an die Target-DNA zu gewährleisten (konstante Annealing-Temperatur von 53°C). Daraufhin wurde eine Temperatur von 72°C für 90 s zur Primer-Extension eingestellt, da hier das Aktivitätsoptimum der verwendeten Polymerase liegt. Schließlich wurden die Ansätze für 10 min bei 72°C inkubiert und bis zur Entnahme aus dem Gerät bei 4°C gekühlt.

### 2. Expression

Das Zielgen wurde nach Amplifikation mittels Polymerasekettenreaktion über die Schnittstellen Ndel und BamHI in den Expressionsvektor pET22b+ einkloniert, in *E. coli* Rosetta DE3™ und *E. coli* BL21 DE3 Zellen eingebracht und exprimiert. Es handelt sich hierbei um nicht-pathogene Stämme, die die Produktion großer Mengen des Enzyms ermöglichen (bis zu 150000 U/I Kultur).

Zur Expression wurden 5 ml LB-Medium (mit 100 µg/ml Ampicillin) mit dem E. coli BL21 (DE)- oder Rosetta™-Klon, der mit einem Expressionsvektor transformiert wurde, für 16 h bei 37°C und 180 rpm inkubiert. 200 ml TB-Medium (mit 100 µg/ml Ampicillin) wurde damit inokuliert und bei 37°C und 180 rpm inkubiert. Bei einer OD₆₀₀ von 0,6-0,8 wurde die Expression von 12α-HSDH mit 1 mM IPTG induziert. Zu verschiedenen Zeitpunkten wurde 1 ml Zellsuspension mit OD₆₀₀ von 0,25 entnommen, für 1 min bei 13000 rpm pelletiert und bis zur weiteren Verwendung bei -20°C gelagert. Die Expression wurde nach 4 bzw. 22 h beendet, indem die Zellen bei 2700 g für 10 min bei 4°C pelletiert und anschließend eingefroren wurden.

Die Zellen wurden mit Hilfe von Ultraschall aufgeschlossen, indem das Pellet zunächst in 4-10 ml Kaliumphosphatpuffer (50 mM, pH 8,0) resuspendiert wurde. Im Ultraschalldesintegrator der Firma Branson wurden die Zellen bei einer Intensität von 30 % viermal 1 min mit jeweils 2 min Pause im Eisbad behandelt. Anschließend wurden die Zelltrümmer 1 h bei 4220 g und 4°C abzentrifugiert.

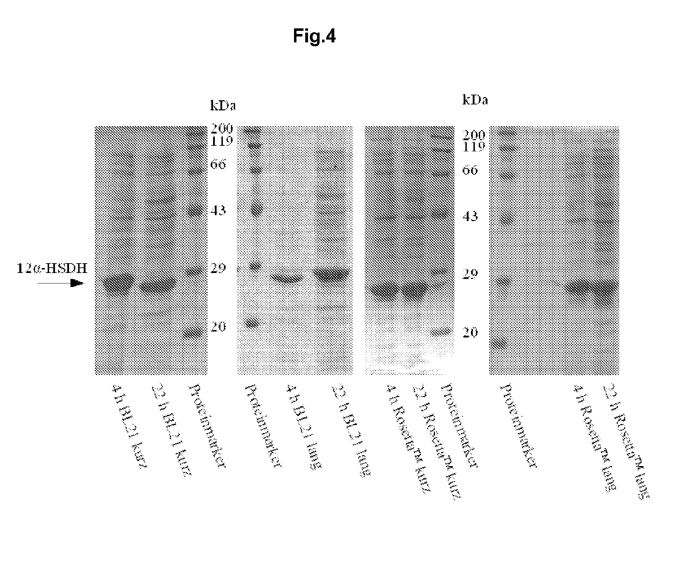

Figur 4 zeigt die Ergebnisse einer SDS-PAGE (12,5%-iges Gel, β-Mercaptoethanol) von Zelllysaten nach 4h und 22h Expression erfindungsgemäßer Enzyme (HSDH_kurz und HSDH_lang) (Bande jeweils bei etwa 27 kDa).

Proteingehalte und Enzymaktivitäten wurden wie oben beschrieben bestimmt.

Die erzielten Enzymaktivitäten nach Aufschluss der *E. coli*-Zellen sind in folgender Tabelle zusammengefasst.

| Stamm (E. coli) | Expressionsdauer [h] | Aktivität [U/I Kulturmedium] | | |
|---|---|---|---|---|
| | | HSDH_lang | HSDH_kurz | pET22b-Leervektor |
| Rosetta™ (DE3) | 4 | 3100 | 3400 | 200 |
| | 22 | 19500 | 24300 | 0 |
| BL21 (DE3) | 4 | 12700 | 12800 | - |
| | 22 | 36800 | 33000 | - |

Durch den hohen Expressionslevel ist die volumetrische und spezifische Aktivität der Enzympräparation nach Zellaufschluss und Zentrifugation bereits deutlich höher (36800 U/I Kulturmedium) als die aus *Clostridium sp.* group P strain 48 -50 stammende. Damit kann im Unterschied zu den bisher beschriebenen Verfahren auf die Proteinaufreinigung verzichtet werden.

Der hohe Anteil des Zielproteins an der Gesamtproteinmenge von BL21 (DE3) Zellen wird durch folgende Tabelle veranschaulicht:

| Stamm | Expressionsdauer [h] | Aktivität [U/I Kulturmedium] | | |
|---|---|---|---|---|
| | | HSDH_lang | HSDH_kurz | pET22b-Leervektor |
| Rosetta™ (DE3) | 4 | 3100 | 3400 | 200 |
| | 22 | 19500 | 24300 | 0 |
| BL21 (DE3) | 4 | 12700 | 12800 | - |
| | 22 | 36800 | 33000 | - |

### Beispiel 3: Präparative Synthese von 12-Ketochenodesoxycholsäure aus Cholsäure

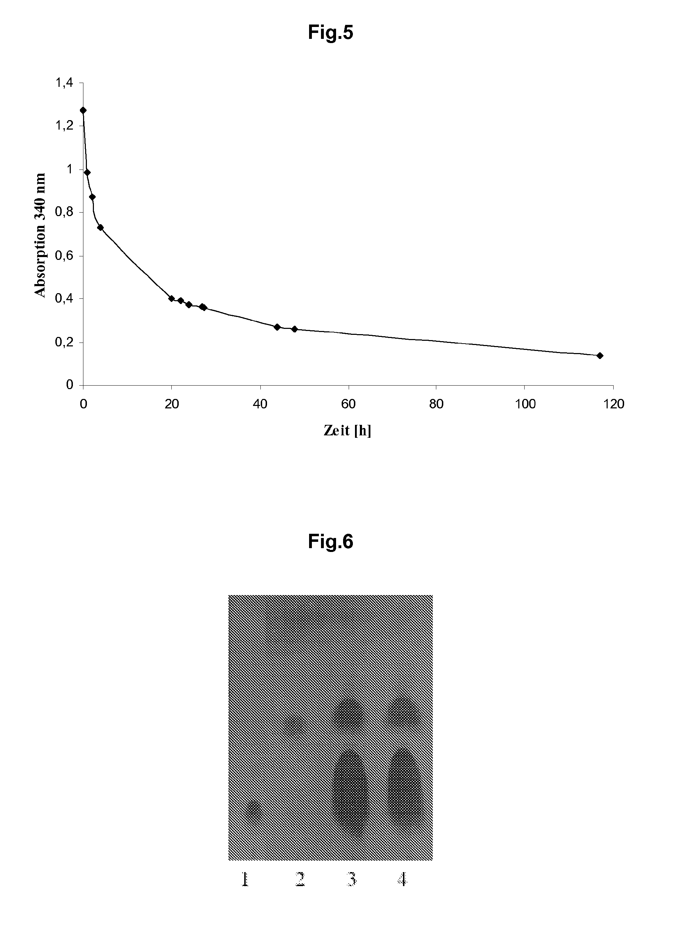

Das exprimierte Enzym (kurze Version) wurde in Kombination mit ADH t (Codexis, Jülich) zur präparativen Synthese von 12-Ketochenodesoxycholsäure eingesetzt. Dazu wurden in einem 1 I-Rundkolben 500 ml Cholsäure (400 mM in Kaliumphosphatpuffer (50 mM, pH 8,0), 10 % Aceton), 0,25 mM NADP⁺, 2000 U 12α-HSDH_kurz aus *E. coli* BL21 (DE 3) (vgl. oben Beispiel 2) und zur Cofaktorregenerierung 550 U ADH t (aus Thermoanaerobacter sp., Codexis, Jülich gemischt. Die Reaktion erfolgte bei RT, unter ständigem Rühren und Rückflusskühlung. Nach 27 h wurden weitere 550 U 12α-HSDH und 138 U ADH t zugegeben und insgesamt 117 h inkubiert. Während der Reaktion wurde die photometrische Absorption bei 340 nm bestimmt und 1 ml Proben zur Verfolgung des Reaktionsverlaufs entnommen, welche mit 100 µl Salzsäure (1 M) abgestoppt und eingedampft bzw. in Essigsäureethylester extrahiert wurden. DerAbsorptionsverlauf ist in Figur 5 dargestellt

Die Reaktion wurde durch Zugabe von rauchender Salzsäure (37 %) bis zur vollständigen Ausfällung der Reaktionspartner angesäuert. Der Überstand wurde abgenommen und mit jeweils 50 ml Essigsäureethylester dreimal extrahiert. Die ausgefallenen Cholsäurederivate wurden in Aceton unter Salzsäurezugabe und Erwärmung vollständig gelöst. Die organischen Phasen wurden vereinigt und bis zur Lösungsmittelfreiheit getrocknet.

Dabei stellte sich heraus, dass die Produktextraktion deutlich einfacher zu bewerkstelligen ist als bei Verwendung des kommerziellen Produkts direkt aus *Clostridium sp.* group P strain 48 -50 (ASA Spezialenzyme). Grund hierfür ist der bei gleicher HSDH-Aktivität deutlich geringere Gesamtproteingehalt.

### Beispiel 4: Charakterisierung der exprimierten Enzyme

Die exprimierten Enzyme wurden hinsichtlich ihrer Aktivität charakterisiert. Es zeigte sich, dass die selektive Oxidation von Cholsäure zu 12-Ketochenodeoxycholsäure katalysiert wird.

Auf eine DC-Alufolie Kiesegel 60 F₂₅₄, Merck, Darmstadt wurden mittels Glaskapillare die Referenzsubstanzen Cholsäure und 12-Ketochenodesoxycholsäure und Extrakte der Reaktionsansätze (Beispiel 3) aufgetragen. Die Folie wurde möglichst senkrecht in eine Chromatografiekammer gestellt, die als Laufmittel ein Gemisch aus Dichlormethan:Aceton:Essigsäure (konz.) im Verhältnis 40:40:3 enthielt, Die Trennung wurde solange durchgeführt, bis die Laufmittelfront fast die Oberkante der Platte erreichte hatte. Die Färbung der Substanzen erfolgte mittels Besprühen mit Molybdatophosphorsäure-Sprühreagens (40 mM Molybdatophosphorsäure, 95,2 % konz. Essigsäure, 4,8 % konz. Schwefelsäure) und anschließender Erhitzung.

Die Ergebnisse sind in Figur 6 dargestellt

### Beispiel 5: Lokalisation von an der NADPH-Bindung beteiligten Aminosäureresten

Über Homologievergleiche mit NADH und NADPH-abhängigen "Short Chain Dehydrogenasen" (SDR) konnten zwei für die Kofaktorerkennung und möglicherweise für eine zukünftige Kofaktordiskriminierung wichtige Aminosäureseitenketten identifiziert werden: Durch ortsgerichtete Mutagenese (Subitutionen wurden basierend auf Publikationen (Tanaka et al. (1996) Biochemistry 35(24): 7715-30.) und (Carugo and Argos (1997) Proteins 28(1): 10-28 erstellt) wurden die Substitutionen G37D und R38L (bezogen auf SEQ ID NO: 3) durchgeführt. Die Experimente wurden nach den experimentellen Angaben zum QuikChange Site-directed Mutagenesis Kit von Stratagene GmbHdurchgeführt.

Die Primer (siehe folgende Tabelle) für die ortsgerichtete Mutagenese wurden auf Grundlage der 12α-HSDH-Gensequenz so gewählt, dass sie den gewünschten Aminosäurenaustausch bewirkten. Dabei wurde darauf geachtet, dass die Mutation (unterstrichen markiert) mittig im Primer lokalisiert war und sich die Schmelztemperatur zweier Primerpaare im gleichen Bereich befand. Es wurden folgende Kombinationen verwendet:
MBr_QC_HSDH_G37D_forw/ MBr_QC_HSDH_G37D_rev mit pET22b(+)-HSDH_kurz
   und
MBr_QC_HSDH_R38L_forw/ MBr_QC_HSDH_R38L_rev mit pET22b(+)-HSDH_kurz_G37D.

### Primer für positionsgerichtete Mutagenese

| **Primer** | **Sequenz** | **Schmelztemperatur** |
|---|---|---|
| MBr_QC_HSDH_G37D_forw | 5′-CTGGTCCTGACCGACAGAAACGAG C-3′ (SEQ ID NO:17) | 63°C |
| MBr_QC_HSDH_G37D_rev | 5´-GCTCGTTTCTGTCGGTCAGGACCA G -3´(SEQ ID NO:18) | 63°C |
| MBr_QC_HSDH_R38L_forw | 5´-GTCCTGACCGACTTAAACGAGCAG AAAC -3´(SEQ ID NO: 19) | 61°C |
| MBr_QC_HSDH_R38L_rev | 5´-GTTTCTGCTCGTTTAAGTCGGTCA GGAC –3´(SEQ ID NO:20 ) | 61°C |

Es ergab sich, dass die resultierenden Proteinvarianten keine Aktivität mehr mit NADPH zeigten. Dies unterstreicht die Wichtigkeit der identifizierten Positionen für die Kofaktorbindung. Die so hergestellten Varianten zeigten bisher keine Aktivität mit NADH. Allerdings könnte durch Sättigungsmutagenese an den beschriebenen oder weiteren Positionen eine NADH-abhängige HSDH-Variante erhalten werden.

Auf die Offenbarung der hierin zitierten Publikationen wird ausdrücklich Bezug genommen.

## Patentansprüche

1. 12α-Hydroxysteroiddehydrogenase (12α-HSDH) erhältlich aus *Clostridium* sp. mit einem Molekulargewicht im Bereich von etwa 27 bis 30 kD.

2. 12α-Hydroxysteroiddehydrogenase nach Anspruch 1, erhältlich aus *Clostridium* sp. group P strain 48-50 (DSM4029).

3. 12α-Hydroxysteroiddehydrogenase nach Anspruch 1 oder 2 mit einer spezifischen Aktivität im Bereich von mehr als etwa 10 U/mg.

4. 12α-Hydroxysteroiddehydrogenase, umfassend wenigstens eines der folgenden Sequenzmotive:
a) LINN (SEQ ID NO:5)
b) RMGIFD (SEQ ID NO: 11)
c) N-terminale Sequenz, ausgewählt unter
(1) MDFIDFKEMGRMGIFDGKVAIITGGGKAKSIGYGIAVAYAK (SEQ ID NO: 6)
(2) MDFIDFKEMGRMGI (SEQ ID NO:7)
(3) ITGGGKAKSIGYGIA (SEQ ID NO:8)
(4) IFDGK (SEQ ID NO: 9)
(5) GIFDGK (SEQ ID NO: 10)
d) FGDPELDI (SEQ ID NO:13).

5. 12α-Hydroxysteroiddehydrogenase,
a) umfassend eine der Aminosäuresequenzen gemäß SEQ ID NO: 2 oder 4, jeweils beginnend bei Position +1 oder +2; oder
b) umfassend eine von einer Sequenz gemäß a) abgeleiteten Aminosäuresequenz mit einer prozentualen Sequenz-Identität von wenigsten 60 %; oder
c) kodiert von einer ein Protein gemäß a) und b) kodierenden Nukleinsäuresequenz; oder
d) kodiert von einer kodierenden Nukleinsäuresequenz gemäß SEQ ID NO: 1 oder 3; oder
e) kodiert von einer der Nukleinsäuresequenzen gemäß SEQ ID NO: 1 oder 3 abgeleiteten kodierenden Sequenz mit einer prozentualen Sequenz-Identität von wenigstens 60 %.

6. 12α-Hydroxysteroiddehydrogenase-Mutante mit modifizierter Cosubstrat-Nutzung.

7. Mutante nach Anspruch 6, abgeleitet von einer 12α-Hydroxysteroiddehydrogenase nach einem der Ansprüche1 bis 5, mit wenigstens einer die Cosubstrat-Nutzung modifizierenden Mutation im Sequenzmotiv VLTGRNE (SEQ ID NO: 12).

8. Mutante nach Anspruch 7, umfassend wenigstens eine der folgenden Aminosäuresubstitutionen in SEQ ID NO:12: G →D ; R →A.

9. 12α-Hydroxysteroiddehydrogenase nach einem der vorhergehenden Ansprüche, erhältlich durch heterologe Expression einer 12α-Hydroxysteroiddehydrogenasekodierenden Nukleinsäuresequenz gemäß Anspruch 5 c), d) oder e).

10. 12α-Hydroxysteroiddehydrogenase nach Anspruch 9, exprimiert in einem nicht-pathogenen Mikroorganismus.

11. 12α-Hydroxysteroiddehydrogenase nach Anspruch 10, exprimiert in einem Bakterium der Gattung Escherichia, insbesondere der Spezies E. coli.

12. Nukleinsäuresequenz gemäß der Definition in Anspruch 5 c), d) oder e).

13. Expressionskassette umfassend eine Nukleinsäuresequenz gemäß Anspruch 12 unter der genetischen Kontrolle wenigstens einer regulativen Nukleinsäuresequenz.

14. Vektor, umfassend wenigstens eine Expressionskassette nach Anspruch 13.

15. Rekombinanter Mikroorganismus, der wenigstens eine Nukleinsäuresequenz gemäß Anspruch 12 oder wenigstens eine Expressionskassette nach Anspruch 13 trägt oder mit wenigstens einem Vektor nach Anspruch 14 transformiert ist.

16. Verfahren zur Herstellung einer 12α-Hydroxysteroiddehydrogenase nach einem der Ansprüche 1 bis 11, wobei man einen Mikroorganismus nach Anspruch 15 kultiviert und die exprimierte 12α-Hydroxysteroiddehydrogenase aus der Kultur isoliert.

17. Verfahren zur enzymatischen Oxidation von 12α-Hydroxysteroiden, wobei man das Hydroxysteroid in Gegenwart einer 12α-Hydroxysteroiddehydrogenase gemäß der Definition in einem der Ansprüche 1 bis 11 umsetzt, und wenigstens ein gebildetes Oxidationsprodukt aus dem Reaktionsansatz gegebenenfalls isoliert.

18. Verfahren nach Anspruch 17, wobei das Hydroxysteroid Cholsäure (CS) oder eine Cholsäurederivat, wie insbesondere ein Salz, Amid oder Alkylester, ist.

19. Verfahren nach Anspruch 18, wobei CS oder ein Derivat davon zu 12-Ketochenodesoxycholsäure (12-Keto-CDCS) oder zum entsprechenden Derivat umgesetzt wird.

20. Verfahren nach einem der Ansprüche 17 bis 19, wobei die Reaktion in Gegenwart von NAD(P)⁺ erfolgt.

21. Verfahren zur enzymatischen Reduktion von 12-Ketosteroiden, wobei man das Ketosteroid in Gegenwart einer 12α-Hydroxysteroiddehydrogenase gemäß der Definition in einem der Ansprüche 1 bis 11 umsetzt, und ein gebildetes Reduktionsprodukt aus dem Reaktionsansatz gegebenenfalls isoliert.

22. Verfahren nach Anspruch 21, wobei das Ketosteroid 12-Keto-CDCS oder ein Derivat davon, wie insbesondere ein Salz, Amid oder Alkylester, ist.

23. Verfahren nach einem der Ansprüche 21 und 22, wobei das Ketosteroid oder dessen Derivat zum korrespondierenden 12α-Hydroxysteroid oder dessen Derivat reduziert wird.

24. Verfahren nach einem der Ansprüche 21 bis 23, wobei die Reaktion in Gegenwart von NAD(P)H erfolgt.

25. Verfahren nach einem der Ansprüche 17 bis 24, wobei die verbrauchten Redox-Äquivalente elektrochemisch oder enzymatisch regeneriert werden.

26. Verfahren nach einem der Ansprüche 17 bis 25, wobei die Reaktion mit einer 12α-Hydroxysteroiddehydrogenase in immobiliserter Form erfolgt.

27. Bioreaktor, umfassend eine 12α-Hydroxysteroiddehydrogenase in immobilisierter Form.

28. Verfahren zum qualitativen oder quantitativen Nachweis von 12-Ketosteroiden bzw. 12α-Hydroxysteroiden, wobei man das Steroid einer durch eine 12α-Hydroxysteroiddehydrogenase nach einem der Ansprüche 1 bis 11 katalysierten Redoxreaktion in Gegenwart von Redoxäquivalenten durchführt, eine Änderung in der Konzentration der Redoxäquivalente bestimmt und daraus den Gehalt an 12-Ketosteroiden bzw. 12α-Hydroxysteroiden qualitativ oder quantitativ ermittelt.

29. Verfahren zur Herstellung von Ursodesoxycholsäure (UDCS) der Formel (1) 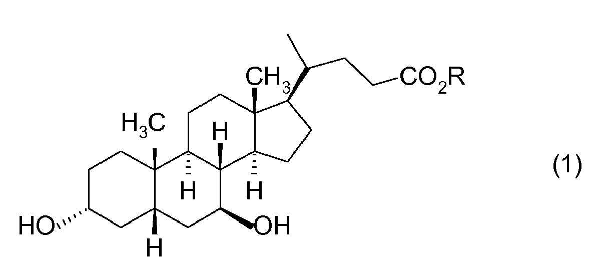 worin
R für Alkyl, NR¹R², H, ein Alkalimetallion oder N(R)₄⁺steht, worin die Reste R³ gleich oder verschieden sind und für H oder Alkyl stehen,
wobei man
a) eine Cholsäure (CS) der Formel (2) 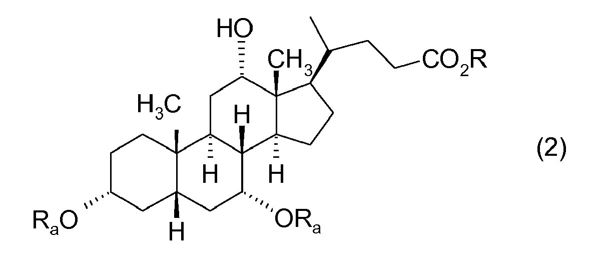 worin R die oben angegebenen Bedeutungen besitzen, und die Reste Rₐgleich oder verschieden sind und für H oder Acyl stehen, in Gegenwart einer 12α-Hydroxysteroiddehydrogenase nach einem der Ansprüche 1 bis 11 zur korrespondierenden 12-Ketochenodesoxycholsäure (12-Keto CDCS) der Formel (3) 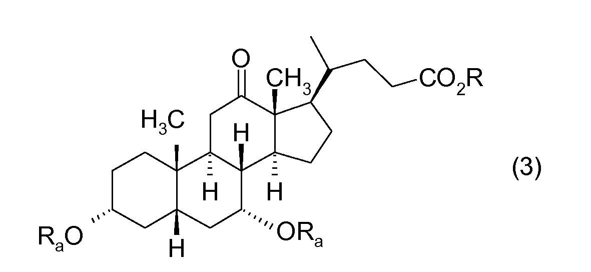 worin R und Rₐ die oben angebebenen Bedeutungen besitzen, oxidiert und anschließend
b) 12-Keto-CDCS der Formel (3) durch Desoxygenierung zu Chenodesoxycholsäure (CDCS) der Formel (4) 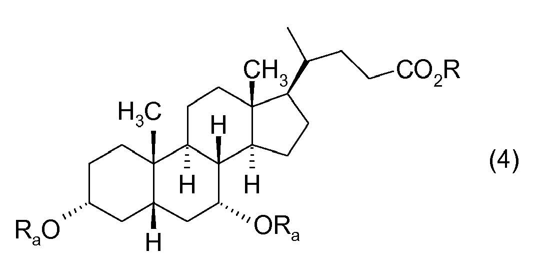 worin R und Rₐ die oben angebebenen Bedeutungen besitzen, umsetzt und
c) CDCS der Formel (4) in Position 7 chemisch oxidiert zur 7-Keto-Lithocholsäure (KLCS) der Formel (5) 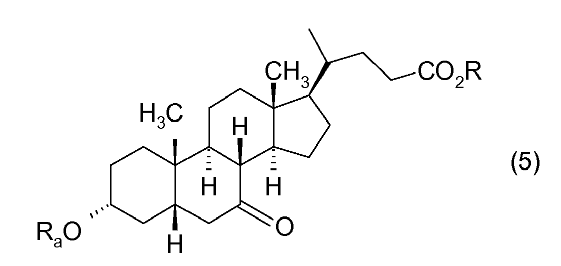 worin R und Rₐ die oben angebebenen Bedeutungen besitzen; und
d) KLCS der Formel (5) reduziert und
e) das Reaktionsprodukt gegebenenfalls weiter aufreinigt.

30. Verfahren nach Anspruch 29, wobei wenn Rₐ für Acyl steht, diese Acylgruppe nach Durchführung der Reaktionsstufe b) oder d) gegebenenfalls abspaltet.

31. Verfahren nach Anspruch 29 oder 30, wobei Stufe a) in Gegenwart von NAD(P)⁺ erfolgt.

32. Verfahren nach Anspruch 31, wobei verbrauchtes NAD(P)⁺ elektrochemisch oder enzymatisch regeneriert werden.

33. Verfahren nach einem der Ansprüche 29 bis 32, wobei Stufe a) mit einer 12a-Hydroxysteroiddehydrogenase in immobilisierter Form erfolgt.


SEQUENCE LISTING

<110>  PHARMAZELL GmbH

<120>  12alpha-HSDH

<130>  M/48429

<160>  20

<170>  PatentIn version 3.3

<210>  1
<211>  813
<212>  DNA
<213>  Clostridium sp.

<220>
<221>  CDS
<222>  (1)..(813)

<400>  1
atg gat ttt att gat ttt aag gag atg ggc aga atg ggg atc ttt gac      48
Met Asp Phe Ile Asp Phe Lys Glu Met Gly Arg Met Gly Ile Phe Asp
1               5                   10                  15

gga aag gtc gca atc att act ggc ggg ggc aag gcc aaa tcg atc ggc      96
Gly Lys Val Ala Ile Ile Thr Gly Gly Gly Lys Ala Lys Ser Ile Gly
            20                  25                  30

tac ggc att gcc gtg gcc tat gct aag gag ggg gcc aac ctg gtc ctg      144
Tyr Gly Ile Ala Val Ala Tyr Ala Lys Glu Gly Ala Asn Leu Val Leu
        35                  40                  45

acc ggc aga aac gag cag aaa ctg ctg gac gcc aag gag gag ctg gag      192
Thr Gly Arg Asn Glu Gln Lys Leu Leu Asp Ala Lys Glu Glu Leu Glu
    50                  55                  60

cgc ctc tac ggc atc aag gtg ttg ccg ctg gcg gtg gac gtc acc ccc      240
Arg Leu Tyr Gly Ile Lys Val Leu Pro Leu Ala Val Asp Val Thr Pro
65                  70                  75                  80

agc gat gag tcg gag gac cgg gtc aag gaa gcc gtg cag aag gtc atc      288
Ser Asp Glu Ser Glu Asp Arg Val Lys Glu Ala Val Gln Lys Val Ile
                85                  90                  95

gcc gaa ttc ggc cgc atc gac gtg ctg atc aac aac gcc cag gcg tcg      336
Ala Glu Phe Gly Arg Ile Asp Val Leu Ile Asn Asn Ala Gln Ala Ser
            100                 105                 110

gcc tcg ggc atc ccc ctg tcc atg cag acc aaa gac cac ttt gac ctg      384
Ala Ser Gly Ile Pro Leu Ser Met Gln Thr Lys Asp His Phe Asp Leu
        115                 120                 125

ggc atc tac tcc ggg ctc tac gcc acc ttc tac tac atg agg gag tgc      432
Gly Ile Tyr Ser Gly Leu Tyr Ala Thr Phe Tyr Tyr Met Arg Glu Cys
    130                 135                 140

tat ccc tac ctg aag gag acc cag ggc tcg gtc atc aac ttc gcc tcc      480
Tyr Pro Tyr Leu Lys Glu Thr Gln Gly Ser Val Ile Asn Phe Ala Ser
145                 150                 155                 160

ggc gcc ggc ctc ttc ggc aac gtg ggt cag tgc tcc tac gcc gcc gcc      528
Gly Ala Gly Leu Phe Gly Asn Val Gly Gln Cys Ser Tyr Ala Ala Ala
                165                 170                 175

aaa gag ggc atc cgc ggc ctc tcc cgc gtc gcg gcc acc gag tgg ggc      576

Lys Glu Gly Ile Arg Gly Leu Ser Arg Val Ala Ala Thr Glu Trp Gly
            180                 185                 190

aag gac aac atc aac gtc aac gtg gtc tgc ccc ctg gcc atg acc gcc 624
Lys Asp Asn Ile Asn Val Asn Val Val Cys Pro Leu Ala Met Thr Ala
        195                 200                 205

cag ctg gag aac ttc aag ctc tcc tac cct gag gcc tac gag aaa aac 672
Gln Leu Glu Asn Phe Lys Leu Ser Tyr Pro Glu Ala Tyr Glu Lys Asn
    210                 215                 220

ctc aga ggg gtg ccc atg ggc cgc ttc ggt gac ccc gag ctg gac atc 720
Leu Arg Gly Val Pro Met Gly Arg Phe Gly Asp Pro Glu Leu Asp Ile
225                 230                 235                 240

ggc cgg gtc tgc gtg cag ctc ggc tcg ccc gac ttc aag tac atg tcc 768
Gly Arg Val Cys Val Gln Leu Gly Ser Pro Asp Phe Lys Tyr Met Ser
                245                 250                 255

ggc gag acc ctc acc ctg gaa ggc ggc atg ggt cag cgc ccc tag 813
Gly Glu Thr Leu Thr Leu Glu Gly Gly Met Gly Gln Arg Pro
            260                 265                 270

<210> 2
<211> 270
<212> PRT
<213> Clostridium sp.

<400> 2

Met Asp Phe Ile Asp Phe Lys Glu Met Gly Arg Met Gly Ile Phe Asp
1               5                   10                  15

Gly Lys Val Ala Ile Ile Thr Gly Gly Gly Lys Ala Lys Ser Ile Gly
            20                  25                  30

Tyr Gly Ile Ala Val Ala Tyr Ala Lys Glu Gly Ala Asn Leu Val Leu
        35                  40                  45

Thr Gly Arg Asn Glu Gln Lys Leu Leu Asp Ala Lys Glu Glu Leu Glu
    50                  55                  60

Arg Leu Tyr Gly Ile Lys Val Leu Pro Leu Ala Val Asp Val Thr Pro
65                  70                  75                  80

Ser Asp Glu Ser Glu Asp Arg Val Lys Glu Ala Val Gln Lys Val Ile
                85                  90                  95

Ala Glu Phe Gly Arg Ile Asp Val Leu Ile Asn Asn Ala Gln Ala Ser
            100                 105                 110

Ala Ser Gly Ile Pro Leu Ser Met Gln Thr Lys Asp His Phe Asp Leu
        115                 120                 125

Gly Ile Tyr Ser Gly Leu Tyr Ala Thr Phe Tyr Tyr Met Arg Glu Cys
    130                 135                 140

Tyr Pro Tyr Leu Lys Glu Thr Gln Gly Ser Val Ile Asn Phe Ala Ser

145                 150                 155                 160

Gly Ala Gly Leu Phe Gly Asn Val Gly Gln Cys Ser Tyr Ala Ala Ala
                165                 170                 175

Lys Glu Gly Ile Arg Gly Leu Ser Arg Val Ala Ala Thr Glu Trp Gly
            180                 185                 190

Lys Asp Asn Ile Asn Val Asn Val Val Cys Pro Leu Ala Met Thr Ala
        195                 200                 205

Gln Leu Glu Asn Phe Lys Leu Ser Tyr Pro Glu Ala Tyr Glu Lys Asn
    210                 215                 220

Leu Arg Gly Val Pro Met Gly Arg Phe Gly Asp Pro Glu Leu Asp Ile
225                 230                 235                 240

Gly Arg Val Cys Val Gln Leu Gly Ser Pro Asp Phe Lys Tyr Met Ser
                245                 250                 255

Gly Glu Thr Leu Thr Leu Glu Gly Gly Met Gly Gln Arg Pro
            260                 265                 270

<210>  3
<211>  774
<212>  DNA
<213>  Artificial

<220>
<223>  Künstliche Kurzsequenz

<220>
<221>  CDS
<222>  (1)..(774)

<400>  3
atc ttt gac gga aag gtc gca atc att act ggc ggg ggc aag gcc aaa      48
Ile Phe Asp Gly Lys Val Ala Ile Ile Thr Gly Gly Gly Lys Ala Lys
1               5                   10                  15

tcg atc ggc tac ggc att gcc gtg gcc tat gct aag gag ggg gcc aac      96
Ser Ile Gly Tyr Gly Ile Ala Val Ala Tyr Ala Lys Glu Gly Ala Asn
            20                  25                  30

ctg gtc ctg acc ggc aga aac gag cag aaa ctg ctg gac gcc aag gag      144
Leu Val Leu Thr Gly Arg Asn Glu Gln Lys Leu Leu Asp Ala Lys Glu
        35                  40                  45

gag ctg gag cgc ctc tac ggc atc aag gtg ttg ccg ctg gcg gtg gac      192
Glu Leu Glu Arg Leu Tyr Gly Ile Lys Val Leu Pro Leu Ala Val Asp
    50                  55                  60

gtc acc ccc agc gat gag tcg gag gac cgg gtc aag gaa gcc gtg cag      240
Val Thr Pro Ser Asp Glu Ser Glu Asp Arg Val Lys Glu Ala Val Gln
65                  70                  75                  80

aag gtc atc gcc gaa ttc ggc cgc atc gac gtg ctg atc aac aac gcc      288
Lys Val Ile Ala Glu Phe Gly Arg Ile Asp Val Leu Ile Asn Asn Ala
                85                  90                  95

cag gcg tcg gcc tcg ggc atc ccc ctg tcc atg cag acc aaa gac cac 336
Gln Ala Ser Ala Ser Gly Ile Pro Leu Ser Met Gln Thr Lys Asp His
            100                 105                 110

ttt gac ctg ggc atc tac tcc ggg ctc tac gcc acc ttc tac tac atg 384
Phe Asp Leu Gly Ile Tyr Ser Gly Leu Tyr Ala Thr Phe Tyr Tyr Met
        115                 120                 125

agg gag tgc tat ccc tac ctg aag gag acc cag ggc tcg gtc atc aac 432
Arg Glu Cys Tyr Pro Tyr Leu Lys Glu Thr Gln Gly Ser Val Ile Asn
    130                 135                 140

ttc gcc tcc ggc gcc ggc ctc ttc ggc aac gtg ggt cag tgc tcc tac 480
Phe Ala Ser Gly Ala Gly Leu Phe Gly Asn Val Gly Gln Cys Ser Tyr
145                 150                 155                 160

gcc gcc gcc aaa gag ggc atc cgc ggc ctc tcc cgc gtc gcg gcc acc 528
Ala Ala Ala Lys Glu Gly Ile Arg Gly Leu Ser Arg Val Ala Ala Thr
                165                 170                 175

gag tgg ggc aag gac aac atc aac gtc aac gtg gtc tgc ccc ctg gcc 576
Glu Trp Gly Lys Asp Asn Ile Asn Val Asn Val Val Cys Pro Leu Ala
            180                 185                 190

atg acc gcc cag ctg gag aac ttc aag ctc tcc tac cct gag gcc tac 624
Met Thr Ala Gln Leu Glu Asn Phe Lys Leu Ser Tyr Pro Glu Ala Tyr
        195                 200                 205

gag aaa aac ctc aga ggg gtg ccc atg ggc cgc ttc ggt gac ccc gag 672
Glu Lys Asn Leu Arg Gly Val Pro Met Gly Arg Phe Gly Asp Pro Glu
    210                 215                 220

ctg gac atc ggc cgg gtc tgc gtg cag ctc ggc tcg ccc gac ttc aag 720
Leu Asp Ile Gly Arg Val Cys Val Gln Leu Gly Ser Pro Asp Phe Lys
225                 230                 235                 240

tac atg tcc ggc gag acc ctc acc ctg gaa ggc ggc atg ggt cag cgc 768
Tyr Met Ser Gly Glu Thr Leu Thr Leu Glu Gly Gly Met Gly Gln Arg
                245                 250                 255

ccc tag 774
Pro

<210> 4
<211> 257
<212> PRT
<213> Artificial

<220>
<223> Synthetic Construct

<400> 4

Ile Phe Asp Gly Lys Val Ala Ile Ile Thr Gly Gly Gly Lys Ala Lys
1               5                   10                  15

Ser Ile Gly Tyr Gly Ile Ala Val Ala Tyr Ala Lys Glu Gly Ala Asn
            20                  25                  30

Leu Val Leu Thr Gly Arg Asn Glu Gln Lys Leu Leu Asp Ala Lys Glu
        35                  40                  45

Glu Leu Glu Arg Leu Tyr Gly Ile Lys Val Leu Pro Leu Ala Val Asp
    50                  55                  60

Val Thr Pro Ser Asp Glu Ser Glu Asp Arg Val Lys Glu Ala Val Gln
65                  70                  75                  80

Lys Val Ile Ala Glu Phe Gly Arg Ile Asp Val Leu Ile Asn Asn Ala
                85                  90                  95

Gln Ala Ser Ala Ser Gly Ile Pro Leu Ser Met Gln Thr Lys Asp His
            100                 105                 110

Phe Asp Leu Gly Ile Tyr Ser Gly Leu Tyr Ala Thr Phe Tyr Tyr Met
        115                 120                 125

Arg Glu Cys Tyr Pro Tyr Leu Lys Glu Thr Gln Gly Ser Val Ile Asn
    130                 135                 140

Phe Ala Ser Gly Ala Gly Leu Phe Gly Asn Val Gly Gln Cys Ser Tyr
145                 150                 155                 160

Ala Ala Ala Lys Glu Gly Ile Arg Gly Leu Ser Arg Val Ala Ala Thr
                165                 170                 175

Glu Trp Gly Lys Asp Asn Ile Asn Val Asn Val Val Cys Pro Leu Ala
            180                 185                 190

Met Thr Ala Gln Leu Glu Asn Phe Lys Leu Ser Tyr Pro Glu Ala Tyr
        195                 200                 205

Glu Lys Asn Leu Arg Gly Val Pro Met Gly Arg Phe Gly Asp Pro Glu
    210                 215                 220

Leu Asp Ile Gly Arg Val Cys Val Gln Leu Gly Ser Pro Asp Phe Lys
225                 230                 235                 240

Tyr Met Ser Gly Glu Thr Leu Thr Leu Glu Gly Gly Met Gly Gln Arg
                245                 250                 255

Pro


<210>  5
<211>  4
<212>  PRT
<213>  Artificial

<220>
<223>  Teilsequenz

<400>  5

Leu Ile Asn Asn
1

<210>  6
<211>  41
<212>  PRT
<213>  Artificial

<220>
<223>  N-terminale Teilsequenz Langversion

<400>  6

Met Asp Phe Ile Asp Phe Lys Glu Met Gly Arg Met Gly Ile Phe Asp
1               5                   10                  15

Gly Lys Val Ala Ile Ile Thr Gly Gly Gly Lys Ala Lys Ser Ile Gly
            20                  25                  30

Tyr Gly Ile Ala Val Ala Tyr Ala Lys
        35                  40

<210>  7
<211>  14
<212>  PRT
<213>  Artificial

<220>
<223>  Nterminale Teilsequenz Langversion

<400>  7

Met Asp Phe Ile Asp Phe Lys Glu Met Gly Arg Met Gly Ile
1               5                   10

<210>  8
<211>  15
<212>  PRT
<213>  Artificial

<220>
<223>  Nterrminale Teilsequenz Kurzversion

<400>  8

Ile Thr Gly Gly Gly Lys Ala Lys Ser Ile Gly Tyr Gly Ile Ala
1               5                   10                  15

<210>  9
<211>  5
<212>  PRT
<213>  Artificial

<220>
<223>  Nterminale Teilsequenz Kurzversion

<400>  9

Ile Phe Asp Gly Lys
1               5

<210>  10
<211>  6

<212>  PRT
<213>  Artificial

<220>
<223>  Nterminale Teilsequenz Kurzversion

<400>  10

Gly Ile Phe Asp Gly Lys
1               5


<210>  11
<211>  6
<212>  PRT
<213>  Artificial

<220>
<223>  Teilsequenz 2

<400>  11

Arg Met Gly Ile Phe Asp
1               5


<210>  12
<211>  7
<212>  PRT
<213>  Artificial

<220>
<223>  Teilsequenzmotiv

<400>  12

Val Leu Thr Gly Arg Asn Glu
1               5


<210>  13
<211>  8
<212>  PRT
<213>  Artificial

<220>
<223>  Cterminale Teilsequenz

<400>  13

Phe Gly Asp Pro Glu Leu Asp Ile
1               5


<210>  14
<211>  37
<212>  DNA
<213>  Artificial

<220>
<223>  PCR Primer forward lang

<400>  14
ggtattccat atggatttta ttgattttaa ggagatg                                37


<210>  15
<211>  33

<212>  DNA
<213>  Artificial

<220>
<223>  PCR Primer Forward kurz

<400>  15
ggtattccat atgatctttg acggaaaggt cgc                                    33


<210>  16
<211>  24
<212>  DNA
<213>  Artificial

<220>
<223>  PCR Primer Revers

<400>  16
cgggatccct aggggcgctg accc                                              24


<210>  17
<211>  25
<212>  DNA
<213>  Artificial

<220>
<223>  PCR Primer

<400>  17
ctggtcctga ccgacagaaa cgagc                                             25


<210>  18
<211>  25
<212>  DNA
<213>  Artificial

<220>
<223>  PCR Primer

<400>  18
gctcgtttct gtcggtcagg accag                                             25


<210>  19
<211>  28
<212>  DNA
<213>  Artificial

<220>
<223>  PCR Primer

<400>  19
gtcctgaccg acttaaacga gcagaaac                                          28


<210>  20
<211>  28
<212>  DNA
<213>  Artificial

<220>
<223>  PCR Primer

<400>  20
gtttctgctc gtttaagtcg gtcaggac                                          28